# NEW EUROPEAN PATENT SPECIFICATION

(11) **EP 3 785 536 B2**
(45) Date of publication and mention of the opposition decision: **17.09.2025**
(45) Mention of the grant of the patent: 26.01.2022
(21) Application number: 19194093.1
(22) Date of filing: 28.08.2019
(51) Int. Cl.: A01K 67/027, C07K 16/00, C12N 9/64

(54) **ADAM6 KNOCKIN MICE**
ADAM6-KNOCKIN-MÄUSE
SOURIS KNOCK-IN À ADAM6

(43) Date of publication of application: 03.03.2021
(73) Proprietor: Trianni, Inc., Wilmington, DE 19801 (US)
(72) Inventor: ESPOSITO, Gloria, 1060 Vienna (AT); WABL, Matthias, San Francisco, CA 94122 (US); MUELLER, Werner, 50931 Köln (DE)
(74) Representative: Redl, Gerda

(56) References cited:
- WO-A1-2012/141798
- WO-A1-2013/079953
- WO-A1-2017/201476
- US-A1- 2017 306 352
- L. E. MACDONALD ET AL: "Precise and in situ genetic humanization of 6 Mb of mouse immunoglobulin genes", PROCEEDINGS OF THE NATIONAL ACADEMY OF SCIENCES, vol. 111, no. 14, 25 March 2014 (2014-03-25), pages 5147 - 5152, XP055126064, ISSN: 0027-8424, DOI: 10.1073/pnas.1323896111
- E-CHIANG LEE ET AL: "Complete humanization of the mouse immunoglobulin loci enables efficient therapeutic antibody discovery", NATURE BIOTECHNOLOGY, vol. 32, no. 4, 16 March 2014 (2014-03-16), New York, pages 356 - 363, XP055131583, ISSN: 1087-0156, DOI: 10.1038/nbt.2825

### SEQUENCE LISTING

<110> Trianni, Inc.
<120> ADAM6 KNOCKIN MICE
<130> TI107EP
<160> 25
<170> PatentIn version 3.5
<210> 1
   <211> 754
   <212> PRT
   <213> Mus musculus
<400> 1
   Met Leu Ser Leu Thr Trp Gly Met Arg Leu Val Glu Arg Pro Val Val
   1               5                   10                  15
   Pro Arg Val Leu Leu Leu Leu Phe Ala Leu Trp Leu Leu Leu Leu Val
               20                  25                  30
   Pro Val Trp Cys Ser Gln Gly His Pro Thr Trp Arg Tyr Ile Ser Ser
           35                  40                  45
   Glu Val Val Ile Pro Arg Lys Glu Ile Tyr His Thr Lys Gly Leu Gln
       50                  55                  60
   Ala Gln Arg Leu Leu Ser Tyr Ser Leu Arg Phe Arg Gly Gln Arg His
   65                  70                  75                  80
   Ile Ile His Leu Arg Arg Lys Thr Leu Ile Trp Pro Arg His Leu Leu
                   85                  90                  95
   Leu Thr Thr Gln Asp Asp Gln Gly Ala Leu Gln Met Glu Tyr Pro Phe
               100                 105                 110
   Phe Pro Val Asp Cys Tyr Tyr Ile Gly Tyr Leu Glu Gly Ile Leu Gln
           115                 120                 125
   Ser Met Val Thr Val Asp Thr Cys Tyr Gly Gly Leu Ser Gly Val Ile
       130                 135                 140
   Lys Leu Asp Asn Leu Thr Tyr Glu Ile Lys Pro Leu Asn Asp Ser Gln
   145                 150                 155                 160
   Ser Phe Glu His Leu Val Ser Gln Ile Val Ser Glu Ser Asp Asp Thr
                   165                 170                 175
   Gly Pro Met Asn Ala Trp Lys His Trp Ser His Asn Thr Gly Ser Pro
   180 185 190
   Ser Ser Arg Leu Glu Tyr Ala Asp Gly Ala Pro Arg Leu Ser Ser Lys
   195 200 205
   Asn Tyr Ala Thr His Pro Ala Ala Ile Lys Gly His Phe Gln Ala Thr
   210 215 220
   His Ser Val Tyr Ser Ala Ser Gly Gly Asp Lys Leu Ser Ser Thr Val
   225 230 235 240
   Glu Tyr Leu Phe Lys Val Ile Ser Leu Met Asp Thr Tyr Leu Thr Asn
   245 250 255
   Leu His Met Arg Tyr Tyr Val Phe Leu Met Thr Val Tyr Thr Glu Ala
   260 265 270
   Asp Pro Phe Ser Gln Asp Phe Arg Val Pro Gly Gly Gln Ala His Thr
   275 280 285
   Phe Tyr Glu Arg Val Phe Tyr Ala His Phe Arg Pro Asp Ala Gly Ala
   290 295 300
   Ile Ile Asn Lys Asn Ser Pro Gly Asp Asp Ala Val Asn Pro Ala Glu
   305 310 315 320
   Arg Ser Ile Cys Ser Pro Ser Ala Leu Ile Cys Leu Gly Gln His Gly
   325 330 335
   Arg Asn Pro Leu Phe Leu Ser Ile Ile Ile Thr Asn Arg Val Gly Arg
   340 345 350
   Ser Leu Gly Leu Lys His Asp Glu Gly Tyr Cys Ile Cys Gln Arg Arg
   355 360 365
   Asn Thr Cys Ile Met Phe Lys Asn Pro Gln Leu Thr Asp Ala Phe Ser
   370 375 380
   Asn Cys Ser Leu Ala Glu Ile Ser Asn Ile Leu Asn Thr Pro Asp Leu
   385 390 395 400
   Met Pro Cys Leu Phe Tyr Asp Arg His Val Tyr Tyr Asn Thr Ser Leu
   405 410 415
   Thr Tyr Lys Phe Cys Gly Asn Phe Lys Val Asp Asn Asn Glu Gln Cys
   420 425 430
   Asp Cys Gly Ser Gln Lys Ala Cys Tyr Ser Asp Pro Cys Cys Gly Asn
           435                 440                 445
   Asp Cys Arg Leu Thr Pro Gly Ser Ile Cys Asp Lys Glu Leu Cys Cys
       450                 455                 460
   Ala Asn Cys Thr Tyr Ser Pro Ser Gly Thr Leu Cys Arg Pro Ile Gln
   465                 470                 475                 480
   Asn Ile Cys Asp Leu Pro Glu Tyr Cys Ser Gly Ser Lys Phe Ile Cys
                   485                 490                 495
   Pro Asp Asp Thr Tyr Leu Gln Asp Gly Thr Pro Cys Ser Glu Glu Gly
               500                 505                 510
   Tyr Cys Tyr Lys Gly Asn Cys Thr Asp Arg Asn Ile Gln Cys Met Glu
           515                 520                 525
   Ile Phe Gly Val Ser Ala Lys Asn Ala Asn Ile Lys Cys Tyr Asp Ile
       530                 535                 540
   Asn Lys Gln Arg Phe Arg Phe Gly His Cys Thr Arg Ala Glu Glu Ser
   545                 550                 555                 560
   Leu Thr Phe Asn Ala Cys Ala Asp Gln Asp Lys Leu Cys Gly Arg Leu
                   565                 570                 575
   Gln Cys Thr Asn Val Thr Asn Leu Pro Phe Leu Gln Glu His Val Ser
               580                 585                 590
   Phe His Gln Ser Val Ile Ser Gly Val Thr Cys Phe Gly Leu Asp Glu
           595                 600                 605
   His Arg Gly Thr Glu Thr Ala Asp Ala Gly Leu Val Arg His Gly Thr
       610                 615                 620
   Pro Cys Ser Arg Gly Lys Phe Cys Asp Arg Gly Ala Cys Asn Gly Ser
   625                 630                 635                 640
   Leu Ser Arg Leu Gly Tyr Asp Cys Thr Pro Glu Lys Cys Asn Phe Arg
                   645                 650                 655
   Gly Val Cys Asn Asn Arg Arg Asn Cys His Cys His Phe Gly Trp Ser
               660                 665                 670
   Pro Pro Lys Cys Lys Glu Glu Gly His Ser Gly Ser Ile Asp Ser Gly
           675                 680                 685
   Ser Pro Pro Val Gln Arg Arg Ile Ile Lys Gln Asn Leu Glu Pro Val
       690                 695                 700
   Val Tyr Leu Arg Ile Leu Phe Gly Arg Ile Tyr Phe Leu Phe Val Ala
   705                 710                 715                 720
   Leu Leu Phe Gly Ile Ala Thr Arg Val Gly Val Thr Lys Ile Phe Arg
                   725                 730                 735
   Phe Glu Asp Leu Gln Ala Ala Leu Arg Ser Trp Gln Glu Gln Ala Lys
               740                 745                 750
   Asp Lys
<210> 2
   <211> 756
   <212> PRT
   <213> Mus musculus
<400> 2
   Met Leu Ser Leu Thr Trp Gly Met Arg Leu Val Glu Arg Pro Val Val
   1               5                   10                  15
   Pro Arg Val Leu Leu Leu Leu Phe Ala Leu Trp Leu Leu Leu Leu Val
               20                  25                  30
   Pro Val Trp Cys Ser Gln Gly His Pro Thr Trp Arg Tyr Ile Ser Ser
           35                  40                  45
   Glu Val Val Ile Pro Arg Lys Glu Ile Tyr His Thr Lys Gly Leu Gln
       50                  55                  60
   Ala Gln Arg Leu Leu Ser Tyr Ser Leu His Phe Arg Gly Gln Arg His
   65                  70                  75                  80
   Ile Ile His Leu Arg Arg Lys Thr Leu Ile Trp Pro Arg His Leu Leu
                   85                  90                  95
   Leu Thr Thr Gln Asp Asp Gln Gly Ala Leu Gln Met Asp Tyr Pro Phe
               100                 105                 110
   Phe Pro Val Asp Cys Tyr Tyr Ile Gly Tyr Leu Glu Gly Ile Pro Gln
           115                 120                 125
   Ser Met Val Thr Val Asp Thr Cys Tyr Gly Gly Leu Ser Gly Val Met
       130                 135                 140
   Lys Leu Asp Asp Leu Thr Tyr Glu Ile Lys Pro Leu Asn Asp Ser Gln
   145 150 155 160
   Ser Phe Glu His Leu Val Ser Gln Ile Val Ser Glu Ser Asp Asp Thr
   165 170 175
   Gly Pro Met Asn Ala Trp Lys His Trp Ser His Asn Thr Gly Ser Pro
   180 185 190
   Ser Ser Arg Leu Glu Tyr Ala Asp Gly Ala Pro Arg Ile Ser Ser Lys
   195 200 205
   Asn Tyr Ala Thr His Pro Ala Ala Ile Lys Gly His Phe Gln Ala Thr
   210 215 220
   Asn Ser Val Tyr Asn Ser Ala Ala Gly Asp Lys Leu Ser Ser Thr Val
   225 230 235 240
   Gly Tyr Leu Phe Gln Val Ile Ser Leu Met Asp Thr Tyr Leu Thr Asn
   245 250 255
   Leu His Met Arg Tyr Tyr Val Phe Leu Met Thr Val Tyr Thr Asn Ser
   260 265 270
   Asp Pro Phe Arg Leu Glu Phe Ala Val Pro Gly Gly Ser Ala Tyr Asn
   275 280 285
   Tyr Tyr Val Ser Val Phe Tyr Asn Lys Phe Lys Pro Asp Ala Gly Val
   290 295 300
   Leu Leu Asn Lys Tyr Gly Pro Gln Asp Asn Gln Val Asn Pro Ala Glu
   305 310 315 320
   Arg Ser Ile Cys Ser Ser Leu Ala Leu Ile Cys Ile Gly Lys Tyr Asp
   325 330 335
   Arg Asn Pro Leu Phe Leu Ser Pro Ile Ile Thr Asn Arg Val Gly Arg
   340 345 350
   Ser Leu Gly Leu Lys Tyr Asp Glu Gly Tyr Cys Val Cys Gln Arg Arg
   355 360 365
   Asn Thr Cys Ile Met Phe Arg His Pro Gln Leu Thr Asp Ala Phe Ser
   370 375 380
   Asn Cys Ser Leu Ala Glu Ile Ser Asn Ile Leu Asn Thr Pro Gly Leu
   385                 390                 395                 400
   Met Pro Cys Leu Phe Tyr Asp Arg His Val Tyr Tyr Asn Thr Ser Leu
                   405                 410                 415
   Thr Tyr Lys Phe Cys Gly Asn Phe Lys Val Asp Asn Asp Glu Gln Cys
               420                 425                 430
   Asp Cys Gly Ser Gln Lys Ala Cys Tyr Ser Asp Pro Cys Cys Gly Asn
           435                 440                 445
   Asp Cys Arg Leu Thr Pro Gly Ser Ile Cys Asp Lys Glu Leu Cys Cys
       450                 455                 460
   Ala Asn Cys Thr Tyr Ser Pro Ser Gly Thr Leu Cys Arg Pro Ile Gln
   465                 470                 475                 480
   Asn Ile Cys Asp Leu Pro Glu Tyr Cys Asn Gly Thr Lys Tyr Ile Cys
                   485                 490                 495
   Pro Asp Asp Thr Tyr Leu Gln Asp Gly Thr Pro Cys Ser Glu Asp Gly
               500                 505                 510
   Tyr Cys Tyr Lys Gly Asn Cys Thr Asp Arg Asn Ile Gln Cys Met Glu
           515                 520                 525
   Ile Phe Gly Val Ser Ala Lys Asn Ala Asn Ile Lys Cys Tyr Asp Ile
       530                 535                 540
   Asn Lys Gln Arg Phe Arg Phe Gly His Cys Thr Arg Ala Glu Glu Ser
   545                 550                 555                 560
   Leu Thr Phe Asn Ala Cys Ala Asp Gln Asp Lys Leu Cys Gly Arg Leu
                   565                 570                 575
   Gln Cys Thr Asn Val Thr Asn Leu Pro Tyr Leu Gln Glu His Val Ser
               580                 585                 590
   Phe His Gln Ser Ile Ile Ser Gly Phe Thr Cys Phe Gly Leu Asp Glu
           595                 600                 605
   His Arg Gly Thr Glu Thr Thr Asp Ala Gly Met Val Arg His Gly Thr
       610                 615                 620
   Pro Cys Ser Lys Ser Lys Phe Cys Asp Gln Gly Ala Cys Ser Gly Ser
   625                 630                 635                 640
   Leu Ser His Leu Gly Tyr Asp Cys Thr Pro Glu Lys Cys Ser Phe Arg
                   645                 650                 655
   Gly Val Cys Asn Asn His Arg Asn Cys His Cys His Phe Gly Trp Lys
               660                 665                 670
   Pro Pro Glu Cys Lys Glu Glu Gly Leu Ser Gly Ser Ile Asp Ser Gly
           675                 680                 685
   Ser Pro Pro Val Gln Arg His Thr Ile Lys Gln Lys Gln Glu Pro Val
       690                 695                 700
   Val Tyr Leu Arg Ile Leu Phe Gly Arg Ile Tyr Phe Leu Phe Val Ala
   705                 710                 715                 720
   Leu Leu Phe Gly Ile Ala Thr Arg Val Gly Val Thr Lys Ile Phe Arg
                   725                 730                 735
   Phe Glu Asp Leu Gln Ala Thr Leu Arg Ser Gly Gln Gly Pro Ala Arg
               740                 745                 750
   Asp Lys Pro Lys
           755
<210> 3
   <211> 2507
   <212> DNA
   <213> Mus musculus
<400> 3
   ggtaaccact taggttggga gcctgaggag gtttttagtc ctctgtcagt attttctaga        60
   ccaggcagtg gccacagcag agggagttag ctccacccag gatgttatct ctgacctggg       120
   gcatgaggct agtggaaaga cctgtggtcc ccagggtcct cctcttgcta tttgcactct       180
   ggctgctcct cctggttcca gtctggtgtt ctcaaggcca tcccacttgg cgttacatct       240
   catcggaggt ggttattcct cggaaggaga tctaccatac caaaggactt caagcacaaa       300
   gactgctctc gtatagcttg cgttttcggg gccagagaca tatcatccac ctgcggagaa       360
   agacactcat ttggcccaga cacttgttgc tgacaactca agatgaccaa ggagccttac       420
   agatggagta cccctttttt cctgtagatt gttactatat tggctacctg gaggggatcc       480
   tgcaatccat ggtcactgtg gatacttgtt atgggggcct gtcaggggtc ataaagttgg       540
   ataaccttac ctatgaaatc aaacccctca atgattcaca gagctttgaa caccttgttt       600
   ctcagatagt atctgagtct gatgacacag ggcctatgaa tgcatggaag cactggagcc       660
   ataatacagg ttctccctcc tccagattgg aatatgcaga tggagctccc agactatcta       720
   gtaagaatta cgctacacat ccagctgcta taaaaggcca ctttcaagca acccattctg       780
   tatatagtgc ttctggaggt gacaaacttt catctactgt tgagtatttg tttaaagtca 840
   ttagtttaat ggacacctat ctgaccaatc ttcatatgcg gtactatgtc tttctcatga 900
   ctgtgtatac cgaggctgat ccattttcac aagattttcg agttccagga gggcaggctc 960
   atactttcta tgagagagta ttttatgctc attttaggcc tgatgcagga gctataatta 1020
   acaagaattc gccaggagat gatgctgtta atccagctga gaggagtata tgttctccct 1080
   cagccctaat ttgtcttggt caacatggtc gaaatccttt atttttatct attataataa 1140
   ccaatcgtgt tggaaggagt ttaggcctaa aacatgatga ggggtactgt atctgccaga 1200
   gaaggaacac ctgcatcatg ttcaaaaatc ctcaattaac agatgctttc agcaattgtt 1260
   cccttgcaga gataagcaac atacttaata ctcctgatct gatgccatgt cttttctatg 1320
   accgtcatgt ttattataat acatcattga cttataagtt ttgtggaaac ttcaaagtag 1380
   ataacaatga gcagtgtgac tgtggctccc aaaaggcatg ttattcagat ccctgctgtg 1440
   gaaatgattg caggttaaca cctggtagca tttgtgataa agaattatgc tgtgcaaatt 1500
   gcacttacag tccttctggg acactctgca gacctatcca gaacatatgt gatcttccag 1560
   agtactgtag tggctctaag ttcatttgcc cagatgacac ttatctgcaa gatgggacac 1620
   catgctcaga agagggttac tgctataaag gtaactgcac tgatcgcaac atacaatgca 1680
   tggaaatctt tggtgtaagt gctaagaatg ctaatattaa gtgctatgac atcaacaaac 1740
   aacggtttcg atttgggcat tgtactagag cagaagaaag cctcacattc aatgcttgtg 1800
   ctgatcagga caagctgtgt ggaaggttgc agtgtaccaa tgtcaccaat cttccatttt 1860
   tgcaagaaca tgtttcattc catcaatcgg ttatctctgg ggttacctgc tttgggcttg 1920
   atgaacatcg tgggacagaa acagcagatg ctggattggt gagacatggt accccgtgtt 1980
   caaggggtaa gttctgtgat cgaggagctt gcaatggaag tttatctcgt ttgggttatg 2040
   actgcacccc agaaaaatgc aatttcagag gagtgtgtaa caatcgtcgg aattgccatt 2100
   gccattttgg ttggagccct ccaaagtgca aagaagaggg acacagtggg agcatagaca 2160
   gtgggtcccc tccggttcaa aggcgcataa taaaacagaa cctagagcca gtagtgtatt 2220
   taagaatact ctttggtcgt atttacttcc tctttgttgc actgctcttt ggcattgcca 2280
   ctcgtgtagg agttactaag atatttaggt ttgaagactt gcaagctgct ttacgttctt 2340
   ggcaagaaca agcaaaggac aagtaaaatg agttagcccc ctgtctcctc taagataaag 2400
   agaatggaaa aaaaatctga atgtcattag gggacaaggt agaactccag tccacagttc 2460
   tggatggcct ttgtttatat catgggacat aagacgtgtt ggactcg 2507
<210> 4
   <211> 2271
   <212> DNA
   <213> Mus musculus
<400> 4
   atgttatctc tgacctgggg catgaggcta gtggaaagac ctgtggtccc cagggtcctc 60
   ctcttgctat ttgcactctg gctgctcctc ctggttccag tctggtgttc tcaaggccat 120
   cctacttggc gttacatctc atcggaggtg gttattcctc ggaaggagat ctaccatacc 180
   aaaggacttc aagcacaaag actgctctca tatagcttgc attttcgggg ccagagacat 240
   atcatccacc tgcggagaaa gacactcatt tggcccagac acttgttgct gacaactcaa 300
   gatgaccaag gagccttaca gatggattac cccttttttc ctgtagattg ttactatatt 360
   ggctacctgg aggggatccc acaatccatg gtcactgtgg atacttgtta tgggggcctg 420
   tcaggggtca tgaagttaga tgaccttacc tatgaaatca aacccctcaa tgattcacag 480
   agctttgaac accttgtttc tcagatagta tctgagtctg atgacacagg gcctatgaat 540
   gcatggaagc actggagcca taatacaggt tctccctcct ccagattgga atatgcagat 600
   ggagctccca gaatatctag taagaactac gctacacatc cagctgctat aaaaggccac 660
   tttcaagcaa ccaattctgt atataattct gctgcaggtg acaaactttc atctactgtt 720
   gggtatttgt ttcaagtcat tagtttaatg gacacctatc tgaccaatct tcatatgcgg 780
   tactatgtct ttctcatgac tgtgtacacc aattctgatc catttcgact tgagtttgca 840
   gttccaggag ggtcggctta taattactat gtgtcagtct tttataataa atttaagcct 900
   gatgcaggag ttttacttaa taagtatggg ccacaagata accaggttaa tccagctgag 960
   aggagtatat gttcttcctt agccctaatt tgtattggta aatatgatcg aaatccttta 1020
   tttttatctc ctataataac caatcgtgtt ggaaggagtt taggcttaaa atatgatgag 1080
   gggtactgtg tctgccagag aaggaacacc tgcattatgt tcagacatcc tcaattaaca 1140
   gatgctttca gcaattgttc ccttgcagag ataagcaaca tacttaatac tcctggtctg 1200
   atgccatgtc ttttctatga ccgtcatgtt tattataata catcattgac ttataagttt 1260
   tgtggaaact tcaaagtaga taacgatgag cagtgtgact gtggctccca aaaggcatgt 1320
   tattcagatc cctgctgtgg aaatgattgc aggttaacac ctggtagcat ttgtgataaa 1380
   gaattatgct gtgcaaattg cacttacagt ccttctggga cactctgcag acctatccag 1440
   aacatatgtg atcttccaga gtactgtaat gggactaaat acatttgccc agatgacact 1500
   tatctgcaag atgggacacc atgctcagaa gatggttact gctataaagg taactgcact 1560
   gatcgcaaca tacaatgcat ggaaatcttt ggtgtaagtg ctaagaatgc taatattaag 1620
   tgctatgaca tcaacaaaca acggtttcga tttgggcatt gtactagagc agaagaaagc 1680
   ctcacattca atgcttgtgc tgatcaggac aagctgtgtg gaaggttgca gtgtaccaat 1740
   gtcaccaatc ttccatattt gcaagaacat gtttcattcc atcaatcgat tatctctggg 1800
   tttacctgct ttgggcttga tgaacatcgt gggacagaaa caacagatgc tggaatggtg 1860
   agacatggta ccccctgctc caaaagtaag ttctgtgatc aaggagcttg cagtggaagt 1920
   ttatctcatt tgggttatga ctgcacccca gaaaaatgca gttttagagg agtgtgtaac 1980
   aatcatcgga attgccattg tcattttggt tggaagcctc cagagtgcaa agaagaggga 2040
   ctaagtggga gcatagacag tgggtcccct ccagttcaaa ggcacacaat aaaacaaaaa 2100
   caagagccag tggtgtattt aagaatactc tttggtcgta tttacttcct ctttgttgca 2160
   ctgctctttg gcattgccac tcgtgtagga gttactaaga tttttagatt tgaagacttg 2220
   caagctactt tacgttctgg gcaaggacca gcaagggaca agccaaagta a 2271
<210> 5
   <211> 200
   <212> DNA
   <213> Artificial Sequence
<220>
   <223> Homology arm sequence
<400> 5
   tgtcctaaag gctctgagat ccctagacag tttatttccc aacttctctc agccggctcc 60
   ctcagggaca aatatccaag attagtctgc aatgctcaga aaactccata acaaaggtta 120
   aaaataaaga cctggagagg ccattcttac ctgaggagac ggtgactgag gttccttgac 180
   cccagtagtc catagcatag 200
<210> 6
   <211> 9777
   <212> DNA
   <213> Artificial Sequence
<220>
   <223> Adam6a and Adam6b and flanking sequences
<400> 6
   aaggcagaag gtcagattca gattcaggta acagggatga cagattctct gttcagtgca 60
   ctcagggtct gcctccacga gaatcaccat gtcctttctc aagactgtgt tctgtgcagt 120
   gccctgtcag tggaaatctg gagagcatgc ttccatgagc ttgtgagtag tatatctagt 180
   aagccatggc tttgtgttaa tggtgatgtt ctacatatca gttctctggc ttaataatga 240
   ggtgatgatt ctatgttcct gtaacgcttc ctcaactggg tcctaagtct ttcttcactc 300
   catctattcc tctaaggaat gatcctgaaa atcccatcac aaactatagg agatgggaac 360
   catcaaaaaa cacagtgaca aagaggtggg aacgcatcag ggttcaggaa ccatatttta 420
   aaaagatatc gtaaataact tcttaaaaga gatatagaca aatctccatt aatacggaga 480
   ccagaggcct aaggctaaga accaatggtg gctcaaggtc tcctgctacc cgaggagcaa 540
   acgtagagca gtttctaatg atttatttaa aatatagaat caaaagtacc agtttgcaat 600
   tttgaaagat ttatttcagc aatgcaacaa catcaggtgg tgccgagtcc aacacgtctt 660
   atgtcccatg atataaacaa aggccatcca gaactgtgga ctggagttct accttgtccc 720
   ctaatgacat tcagattttt tttccattct ctttatctta gaggagacag ggggctaact 780
   cattttactt gtcctttgct tgttcttgcc aagaacgtaa agcagcttgc aagtcttcaa 840
   acctaaatat cttagtaact cctacacgag tggcaatgcc aaagagcagt gcaacaaaga 900
   ggaagtaaat acgaccaaag agtattctta aatacactac tggctctagg ttctgtttta 960
   ttatgcgcct ttgaaccgga ggggacccac tgtctatgct cccactgtgt ccctcttctt 1020
   tgcactttgg agggctccaa ccaaaatggc aatggcaatt ccgacgattg ttacacactc 1080
   ctctgaaatt gcatttttct ggggtgcagt cataacccaa acgagataaa cttccattgc 1140
   aagctcctcg atcacagaac ttaccccttg aacacggggt accatgtctc accaatccag 1200
   catctgctgt ttctgtccca cgatgttcat caagcccaaa gcaggtaacc ccagagataa 1260
   ccgattgatg gaatgaaaca tgttcttgca aaaatggaag attggtgaca ttggtacact 1320
   gcaaccttcc acacagcttg tcctgatcag cacaagcatt gaatgtgagg ctttcttctg 1380
   ctctagtaca atgcccaaat cgaaaccgtt gtttgttgat gtcatagcac ttaatattag 1440
   cattcttagc acttacacca aagatttcca tgcattgtat gttgcgatca gtgcagttac 1500
   ctttatagca gtaaccctct tctgagcatg gtgtcccatc ttgcagataa gtgtcatctg 1560
   ggcaaatgaa cttagagcca ctacagtact ctggaagatc acatatgttc tggataggtc 1620
   tgcagagtgt cccagaagga ctgtaagtgc aatttgcaca gcataattct ttatcacaaa 1680
   tgctaccagg tgttaacctg caatcatttc cacagcaggg atctgaataa catgcctttt 1740
   gggagccaca gtcacactgc tcattgttat ctactttgaa gtttccacaa aacttataag 1800
   tcaatgatgt attataataa acatgacggt catagaaaag acatggcatc agatcaggag 1860
   tattaagtat gttgcttatc tctgcaaggg aacaattgct gaaagcatct gttaattgag 1920
   gatttttgaa catgatgcag gtgttccttc tctggcagat acagtacccc tcatcatgtt 1980
   ttaggcctaa actccttcca acacgattgg ttattataat agataaaaat aaaggatttc 2040
   gaccatgttg accaagacaa attagggctg agggagaaca tatactcctc tcagctggat 2100
   taacagcatc atctcctggc gaattcttgt taattatagc tcctgcatca ggcctaaaat 2160
   gagcataaaa tactctctca tagaaagtat gagcctgccc tcctggaact cgaaaatctt 2220
   gtgaaaatgg atcagcctcg gtatacacag tcatgagaaa gacatagtac cgcatatgaa 2280
   gattggtcag ataggtgtcc attaaactaa tgactttaaa caaatactca acagtagatg 2340
   aaagtttgtc acctccagaa gcactatata cagaatgggt tgcttgaaag tggcctttta 2400
   tagcagctgg atgtgtagcg taattcttac tagatagtct gggagctcca tctgcatatt 2460
   ccaatctgga ggagggagaa cctgtattat ggctccagtg cttccatgca ttcataggcc 2520
   ctgtgtcatc agactcagat actatctgag aaacaaggtg ttcaaagctc tgtgaatcat 2580
   tgaggggttt gatttcatag gtaaggttat ccaactttat gacccctgac aggcccccat 2640
   aacaagtatc cacagtgacc atggattgca ggatcccctc caggtagcca atatagtaac 2700
   aatctacagg aaaaaagggg tactccatct gtaaggctcc ttggtcatct tgagttgtca 2760
   gcaacaagtg tctgggccaa atgagtgtct ttctccgcag gtggatgata tgtctctggc 2820
   cccgaaaacg caagctatac gagagcagtc tttgtgcttg aagtcctttg gtatggtaga 2880
   tctccttccg aggaataacc acctccgatg agatgtaacg ccaagtggga tggccttgag 2940
   aacaccagac tggaaccagg aggagcagcc agagtgcaaa tagcaagagg aggaccctgg 3000
   ggaccacagg tctttccact agcctcatgc cccaggtcag agataacatc ctgggtggag 3060
   ctaactccct ctgctgtggc cactgcctgg tctagaaaat actgacagag gactaaaaac 3120
   ctcctcaggc tcccaaccta agtggttacc cagacaactg gagttaggta acagtcactg 3180
   ggtgtggcag gaattgagtc tgaatgtgtt agctgaggtt gaggttaaat attgtcaaaa 3240
   gggatgtcta taaatgtgcc tggacaagaa aagtcagaag cagcaaggag tgtctctgac 3300
   aggctcaatc ctttcttttc tttttttgaa gttcaaaata tcatttccac gtgaatgtat 3360
   ttggttccca gtgtgactct gggtctcttt ctaggagtca atatttcttt atatcttggc 3420
   tcatgttttt cacagttgtt ctaacttctt gttttgtttt gtttgtttgt ttgtttgaaa 3480
   gttagaagta aatactgtct atattagcct tttagctata aatgattgtt tttatttctt 3540
   ctaatcatgt tttgtttgag ttttggttaa actatttaca aatgagtttt ttttttcctt 3600
   ttgggtgttg ctcgaaagtt tggagctttc tgttaatatt gtgttgttgt ttctccaata 3660
   ttattagacc tgagaattct acctgggtac ctgtgaactc cagaattttt aaaaattcca 3720
   tctcttggga acattatctc tgaccccgtc tgaggccgaa gtggctgtcc ccctccaacc 3780
   tttagtatct ttctttcctg actattggga tttcttcaag caatcaggct gatgggttct 3840
   cagcagtgag accagtagac tgtcggtatg aacgtcgaag agtctgccac acactccggg 3900
   ttcatcaaca gtgctttcgc gtctcttact tttgtagaag gaaatgcagc ctctgagttt 3960
   tctccaagaa atcattgatg aaagggtgaa aagatgggta tcacccggag ttcatgacaa 4020
   gccctggctc agacacgtga gcaaggtcta cagccccaaa gataggctgc cctgcaacat 4080
   gtatttataa gataggagaa aaaaatgggt agttggaggg ttgatcaact tacttcctct 4140
   caaacatata tatctcatct aagtgtgcag gggaaaactc tgtagaacta ctgggatacc 4200
   tgctcacccc caggagcctc atgaataagt ctctgcttct gccttgtagc catgagcatt 4260
   actgcacctg atacccctgc agcttcctag ggaagaggga ggaagtgact tggcccctgt 4320
   ctggttaagg taagaggaga taaatccctt ctcattgatt agggtgagag gggtcatgtg 4380
   ctctatcatt ggtgacccag ttgggacatg ggtttatacc aaagtcatca ctctgaggtt 4440
   ctgtgtacca ccaggctgaa ctcccatatc ctacatggac ataggacaac accaagcaga 4500
   aggaggtttt aggactaaac tgaaggacag agatgcggtt tctaaacaac tagggagtgc 4560
   cagggccagc ctctctaacc actataggac actgtggagt ctggttacaa agagagatta 4620
   ctcaaggtcc ttagcactga ttacagagca tatctcagat gccttctgct gaccagatgt 4680
   atctttgcat aatctgccta tccagattca gaaaattgat gccacatagc caagtggact 4740
   ttcaggaaca gacgatttaa aaacaggcgc tccctctctg cttggacccc cttcctttgg 4800
   ccgggaaggt ggccagatgt ctggctacac taactttctt aataaacttc ctatttataa 4860
   ggaatcagct tatgaaagat gtcacatcaa aatgttgata ctgccagact gtcaggtgct 4920
   gtttaaacag agaagctgac cctgcacctg gaatttgttc cattccactt taaacatagt 4980
   gtttggttca aaaggtcctc acctgggtct taatacctaa agagatgatt gttctactcc 5040
   agaacacact tttcccattg gacaattacc ctccctttat ttaaaaaata gcatgtctct 5100
   ctgcctttat tacacacaca cacacacaca cacacacaca cacacacaca cacactttcc 5160
   tgtcttcatt attttctcat tttagtcctt ctacttagga ccctgtcaat aaaaaaaaaa 5220
   aaatggcctg atggtccaga tcaattggaa ctttataaaa aaggagagga gggaataaat 5280
   gttgttcatg actccctcag ggaaaaattc agtcgttgca catctgtcac agcttttgta 5340
   gctcttgtta gctacagtat gctagatgaa gtcagccttg catacatacc ctgcacacct 5400
   acacatgcaa ggaataaaag aggctaaggt gaaccttaga tctggtttaa acaccagtta 5460
   gtgggcttca gggcctccag agggatgaac tgcagactct caagatgttc tgggagctcc 5520
   acattcacac aaaacaagtc aatccctctt ctctgcttgc ttggtgaccc agtggtagct 5580
   tttgtgttgc cagaactttc tttgcatcgg ttaagaatta ttaggaagtg caggatcaag 5640
   ggcaacagta actatgatgt gtcccaaatt ctgccagtag agggatgtga attatctctt 5700
   gtcaagctag agaaaacctg aggatgaaca gctgagaaag gagtaaggca gactgcagtc 5760
   agcagctaca gacactgtac atcatttttc agttgactaa tatacatcga tgcagctaag 5820
   aaggcagtga tcccaaggaa ggttattggc gttccgaaaa atatatgagc acattaacag 5880
   aacagccctt accccaaact ccttcagaat aaactagttt caaacacagt tgtctagcat 5940
   gtaatcccat cagtctcgtg gaggaagact ctgagtgcat ggaacagaga atctgtcagc 6000
   agtgtaccct gaatctgaat atgaccttct gctataggag aaaggaacca tcaaaaaagc 6060
   acagtgataa tgatgtggga acgcttcagt gttcaggaac catatgattt atttaaaata 6120
   tagaatcaaa agtaccaatt tgcagttttg aaagatttat tccagtgtaa gcattagcaa 6180
   tgcaccaaca tcaggtgatt tctgaatcca acacgtctta tgtcctcatg atattaaaaa 6240
   aaaaaaaagg ccatccagaa ctgtgaactt gagttctacc ttgttcccta ctgacattca 6300
   gattttcttt tttgcattct ctttatctta caggagacag gaggggaggg ctaactcatt 6360
   ttactttggc ttgtcccttg ctggtccttg cccagaacgt aaagtagctt gcaagtcttc 6420
   aaatctaaaa atcttagtaa ctcctacacg agtggcaatg ccaaagagca gtgcaacaaa 6480
   gaggaagtaa atacgaccaa agagtattct taaatacacc actggctctt gtttttgttt 6540
   tattgtgtgc ctttgaactg gaggggaccc actgtctatg ctcccactta gtccctcttc 6600
   tttgcactct ggaggcttcc aaccaaaatg acaatggcaa ttccgatgat tgttacacac 6660
   tcctctaaaa ctgcattttt ctggggtgca gtcataaccc aaatgagata aacttccact 6720
   gcaagctcct tgatcacaga acttactttt ggagcagggg gtaccatgtc tcaccattcc 6780
   agcatctgtt gtttctgtcc cacgatgttc atcaagccca aagcaggtaa acccagagat 6840
   aatcgattga tggaatgaaa catgttcttg caaatatgga agattggtga cattggtaca 6900
   ctgcaacctt ccacacagct tgtcctgatc agcacaagca ttgaatgtga ggctttcttc 6960
   tgctctagta caatgcccaa atcgaaaccg ttgtttgttg atgtcatagc acttaatatt 7020
   agcattctta gcacttacac caaagatttc catgcattgt atgttgcgat cagtgcagtt 7080
   acctttatag cagtaaccat cttctgagca tggtgtccca tcttgcagat aagtgtcatc 7140
   tgggcaaatg tatttagtcc cattacagta ctctggaaga tcacatatgt tctggatagg 7200
   tctgcagagt gtcccagaag gactgtaagt gcaatttgca cagcataatt ctttatcaca 7260
   aatgctacca ggtgttaacc tgcaatcatt tccacagcag ggatctgaat aacatgcctt 7320
   ttgggagcca cagtcacact gctcatcgtt atctactttg aagtttccac aaaacttata 7380
   agtcaatgat gtattataat aaacatgacg gtcatagaaa agacatggca tcagaccagg 7440
   agtattaagt atgttgctta tctctgcaag ggaacaattg ctgaaagcat ctgttaattg 7500
   aggatgtctg aacataatgc aggtgttcct tctctggcag acacagtacc cctcatcata 7560
   ttttaagcct aaactccttc caacacgatt ggttattata ggagataaaa ataaaggatt 7620
   tcgatcatat ttaccaatac aaattagggc taaggaagaa catatactcc tctcagctgg 7680
   attaacctgg ttatcttgtg gcccatactt attaagtaaa actcctgcat caggcttaaa 7740
   tttattataa aagactgaca catagtaatt ataagccgac cctcctggaa ctgcaaactc 7800
   aagtcgaaat ggatcagaat tggtgtacac agtcatgaga aagacatagt accgcatatg 7860
   aagattggtc agataggtgt ccattaaact aatgacttga aacaaatacc caacagtaga 7920
   tgaaagtttg tcacctgcag cagaattata tacagaattg gttgcttgaa agtggccttt 7980
   tatagcagct ggatgtgtag cgtagttctt actagatatt ctgggagctc catctgcata 8040
   ttccaatctg gaggagggag aacctgtatt atggctccag tgcttccatg cattcatagg 8100
   ccctgtgtca tcagactcag atactatctg agaaacaagg tgttcaaagc tctgtgaatc 8160
   attgaggggt ttgatttcat aggtaaggtc atctaacttc atgacccctg acaggccccc 8220
   ataacaagta tccacagtga ccatggattg tgggatcccc tccaggtagc caatatagta 8280
   acaatctaca ggaaaaaagg ggtaatccat ctgtaaggct ccttggtcat cttgagttgt 8340
   cagcaacaag tgtctgggcc aaatgagtgt ctttctccgc aggtggatga tatgtctctg 8400
   gccccgaaaa tgcaagctat atgagagcag tctttgtgct tgaagtcctt tggtatggta 8460
   gatctccttc cgaggaataa ccacctccga tgagatgtaa cgccaagtag gatggccttg 8520
   agaacaccag actggaacca ggaggagcag ccagagtgca aatagcaaga ggaggaccct 8580
   ggggaccaca ggtctttcca ctagcctcat gccccaggtc agagataaca tcctgggtgg 8640
   agctaaatcc ctctgctgtg gccactgcct ggtctagaaa atactgacag aggactaaaa 8700
   acctcctcag gctcccaacc taagtggtta cccagacaac tggagttagg taacagtcac 8760
   tgggtgtggc aggaattgag tctgaatgtg ttagctgagg ttgaggttaa atattgtcaa 8820
   aagggatgtc tataaatgtg cctggacaag aaaagtcaga agcagcaagg agtgtctctg 8880
   acaggctcaa tcctttcttt tctttttttg aagttcaaaa tatcatttcc acgtgaatgt 8940
   atttggttcc cagtgtgact ctgggtctct ttctaggagt caatatttct ttatatcttg 9000
   gctcatgttt ctcacagttg ttctaatttc ttgttttgtt ttgtttgttt gtttgaacgt 9060
   tagtagtaaa tactgtctat attagccttt tagctataaa tgattgtttt tatttcttct 9120
   aatcatattt tgtttgagtt ttggttaaac tatttacaaa tgagtttttt ttttttcctt 9180
   ttgggtgttg ctcgaaagtt tggagctttc tgttaatatt gtgttgttat ttttccaata 9240
   ttattagacc tgagaattct atctgggtac ctgtgaactc tagaattttt aaaaattcca 9300
   tctcttggga acattacctc tgaccccgtc tgaggccgaa gtggctgtcc ccctccaacc 9360
   tttagtatct ttctttcctg actattggga tttcttcaag caatcaggct gatgggttct 9420
   cagcagtgag accagtagac tgccggtatg aacgtcgaag agactgccac acactccagg 9480
   ttcatcaaca gtgctttcgc gtctcttact tttgtagaag gaaaagcagc ctctgagtta 9540
   tctccaagaa atcattaatg aaagagttaa aagatgggta tcacccggag ttcatgacaa 9600
   gccctggctc agacacgtga gcaaggtcta cagccccaaa gataggctgc cctgcaacat 9660
   gtatttataa gatagaagaa aaaaatgggt ggttggaggg ttgatcaact tacttcctct 9720
   caaacatata tatctcatct aagtgtgcag gggaaaactc tgtaggacta ctgggat 9777
<210> 7
   <211> 23
   <212> DNA
   <213> Artificial Sequence
<220>
   <223> TRN0048 Proximal guide RNA (gRNA1)
<400> 7
   gctaaggtga accttagatc tgg 23
<210> 8
   <211> 23
   <212> DNA
   <213> Artificial Sequence
<220>
   <223> TRN0048 Distal guide RNA (gRNA2)
<400> 8
   agagagtata ggaggttccg ggg 23
<210> 9
   <211> 21
   <212> DNA
   <213> Artificial Sequence
<220>
   <223> TRN0048 PCR Oligo 1(forward primer)
<400> 9
   cagtcgttgc acatctgtca c 21
<210> 10
   <211> 20
   <212> DNA
   <213> Artificial Sequence
<220>
   <223> TRN0048 PCR Oligo 2(reverse primer)
<400> 10
   gcctagtagg ccatcaatgg 20
<210> 11
   <211> 22
   <212> DNA
   <213> Artificial Sequence
<220>
   <223> TRN0048 PCR Oligo 3(reverse primer)
<400> 11
   agtgtctgta gctgctgact gc 22
<210> 12
   <211> 23
   <212> DNA
   <213> Artificial Sequence
<220>
   <223> TRN0059 Proximal guide RNA (gRNA3)
<400> 12
   cccagttgag gaagcgttac agg 23
<210> 13
   <211> 23
   <212> DNA
   <213> Artificial Sequence
<220>
   <223> TRN0059 Distal guide RNA (gRNA1
<400> 13
   gctaaggtga accttagatc tgg 23
<210> 14
   <211> 20
   <212> DNA
   <213> Artificial Sequence
<220>
   <223> TRN0059 PCR Oligo 1(forward primer)
<400> 14
   ccctgtcagt ggaaatctgg 20
<210> 15
   <211> 19
   <212> DNA
   <213> Artificial Sequence
<220>
   <223> TRN0059 PCR Oligo 2(reverse primer)
<400> 15
   actgggtcac caagcaagc 19
<210> 16
   <211> 20
   <212> DNA
   <213> Artificial Sequence
<220>
   <223> TRN0059 PCR Oligo 3(reverse primer)
<400> 16
   acctgatgtt gttgcattgc 20
<210> 17
   <211> 20
   <212> DNA
   <213> Artificial Sequence
<220>
   <223> Adam6a Taqman forward primer
<400> 17
   ataccgaggc tgatccattt 20
<210> 18
   <211> 27
   <212> DNA
   <213> Artificial Sequence
<220>
   <223> Adam6a Taqman reverse primer
<400> 18
   ctcctggcga attcttgtta attatag 27
<210> 19
   <211> 24
   <212> DNA
   <213> Artificial Sequence
<220>
   <223> Adam6a Taqman probe
<400> 19
   ccaggagggc aggctcatac tttc 24
<210> 20
   <211> 21
   <212> DNA
   <213> Artificial Sequence
<220>
   <223> Adam6b Taqman forward primer
<400> 20
   tcgacttgag tttgcagttc c 21
<210> 21
   <211> 22
   <212> DNA
   <213> Artificial Sequence
<220>
   <223> Adam6b Taqman reverse primer
<400> 21
   ctggttatct tgtggcccat ac 22
<210> 22
   <211> 30
   <212> DNA
   <213> Artificial Sequence
<220>
   <223> Adam6b Taqman probe
<400> 22
   tgacacatag taattataag ccgaccctcc 30
<210> 23
   <211> 200
   <212> DNA
   <213> Artificial Sequence
<220>
   <223> Homology arm sequence
<400> 23
   ggtcctgaag tatattccac aactaatact tttattctaa aaactgaaaa tctccaacta      60
   cagccccaac tatccctcca gccataggat tgttttagca tcttcttcaa atgagcctcc     120
   aaagtcccta tcccatcatc cagggactcc accaacacca tcacacagat tcttagtttt     180
   tcaaagatgt ggagataatc                                                 200
<210> 24
   <211> 200
   <212> DNA
   <213> Artificial Sequence
<220>
   <223> Homology arm sequence
<400> 24
   ctatgaatat gtacatttag atatatctga aactgagaaa cccagtttga gtttctcatg      60
   ccatcaatgc caactatatc atgcagtata agactaatca ggctaatgac agtatgcctt     120
   tcacacttgg gatggaggaa gaagcaaaaa gaattgataa tgatggtgac acacaggtcc     180
   acaggctttc ccagcatccc                                                 200
<210> 25
   <211> 200
   <212> DNA
   <213> Artificial Sequence
<220>
   <223> Homology arm sequence
<400> 25
   agggaaatta ttttatcagt aagtatatgt ttaatatttc aaaatagcaa aaactctttg      60
   aagttaaaca ttaagaaaat gctaatttca agcacagtga gaaaattatc aataataatt     120
   ccacgtttgt agaacatgat tttaatattt tcaactgcta atagtctaca aacagattaa     180
   ttattttaag atatatttca                                                 200

## Description

### FIELD OF THE INVENTION

The invention relates to the field of transgenic mice comprising a genetic modification that reduces expression of endogenous *Adam6* genes, which are engineered to restore male fertility. The invention specifically relates to mice comprising an exogenous *Adam6* transgene expressing an ADAM6 protein that is functional in a male mouse. The invention further relates to methods of generating such mice and their use for the production of antibodies, specifically chimeric or humanized antibodies.

### BACKGROUND OF THE INVENTION

Antibodies have emerged as important biological pharmaceuticals because they (i) exhibit exquisite binding properties that can target antigens of diverse molecular forms, (ii) are physiological molecules with desirable pharmacokinetics that make them well tolerated in treated humans and animals, and (iii) are associated with powerful immunological properties that naturally ward off infectious agents. Furthermore, established technologies exist for the rapid isolation of antibodies from laboratory animals, which can readily mount a specific antibody response against virtually any substance not present natively in the body. Monoclonal antibodies (mAbs), which are derived from a single responding B lymphocyte, can be readily isolated as immortalized hybridoma cell lines from laboratory animals, usually mice, responding to a particular antigen. A panel of monoclonal antibodies specific for that antigen can then be characterized for their fine specificity (epitope mapping), potential cross reactivity with other antigens, immunoglobulin heavy chain isotype, etc. and then mAbs with the desired biological properties can be identified.

For therapeutic purposes, ideally the mAbs should be as close as possible in protein sequence to the endogenous immunoglobulins of the species being treated. Otherwise, the mAbs will be recognized by the recipient as foreign and provoke an immune response. Recipient antibodies produced against the therapeutic mAbs will neutralize them, abolishing any long-term therapeutic efficacy.

In the case of human therapeutic antibodies, mice that contain human antibody genes are known in the art and exist in various formats, e.g., Regeneron, US8502018B2.

### Mouse reproduction and ADAM6

Spermatozoa leaving the testes enter a long tubular structure called the epididymis. At this stage they are infertile, but undergo maturation, progressive increase in motility, and ultimately the ability to fertilize the ova as they travel through the epididymis. Although many details are still unknown, maturation involves the gain, loss and modification of spermatozoa proteins, a process involving proteins secreted from epididymal epithelial cells. There are subregions of the epididymis where a different array of proteins is secreted to promote progressive maturation of the spermatozoa. Proteomic studies indicate that approximately one third of the proteins with sperm phenotypes in gene knockout mice are added to the spermatozoa during their transit through the epididymis. After maturation, the spermatozoa are stored in the distal epididymis until they are released at ejaculation into the female mouse uterus. Only a small fraction of the spermatozoa then enters the oviduct via the uterotubal junction (UTJ), the connection between the endometrial cavity of the uterus and the fallopian tube. There is evidence for selection against abnormal sperm, particularly those with abnormal morphology, during passage through the UTJ. The spermatozoa that survive this passage become immobilized in the endosalpinx, the mucous membrane lining the fallopian tube, until ovulation occurs, at which time a few of them at a time are released and migrate to the ampulla of the fallopian tube, where fertilization takes place. Because of the limited number of sperm actually reaching the egg, the sperm-egg ratio at the time of fertilization *in vivo* is close to one. Fertilization requires the sperm acrosome reaction, which is necessary to penetrate the zona pellucida, the glycoprotein layer surrounding the egg plasma membrane, and allow fusion of the sperm and egg membranes. In mice, the zona pellucida sperm-binding protein 3 (ZP3) functions in the initial species-specific sperm binding and in initiating the acrosome reaction.

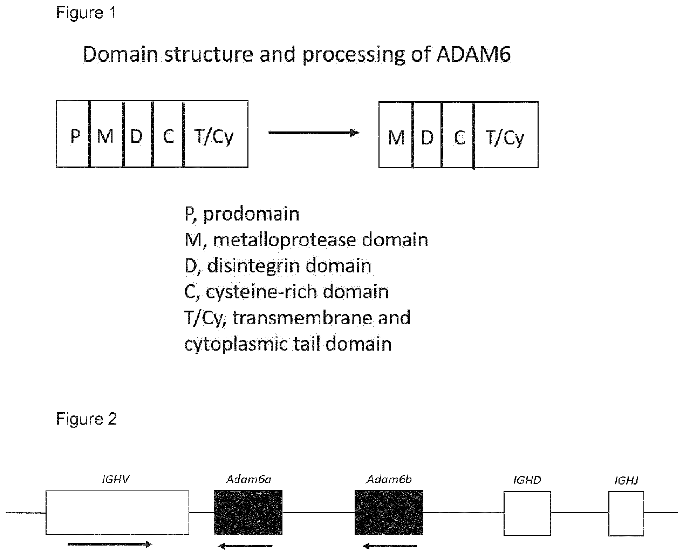

**ADAM6 is part of a large** "A disintegrin and metalloprotease domain-containing **protein"** (ADAM) family comprising 34 known members in mice. More than half of the ADAMs are involved in male reproduction, and the rest are important for other biological processes, e.g. the proteolytic release of the active soluble form of tumor necrosis factor α (TNF-α) from the inactive transmembrane form by TNF-α converting enzyme (TACE or ADAM17). The ADAM family is characterized by a conserved multidomain structure consisting of the prodomain, metalloprotease domain, disintegrin domain, cysteine-rich domain, and a transmembrane and cytoplasmic tail domain (Figure 1). The ADAM6 precursor protein is present on testicular sperm and is processed to remove the prodomain during spermatozoa maturation. The prodomain helps maintain the stability of the protein and occupies the active site of the metalloprotease domain, preventing enzymatic activity. However, the consensus sequence of an active Zn-dependent metalloprotease is not present in ADAM6 and thus it is thought to lack enzymatic activity. The disintegrin domain is involved in binding to integrins. Because of the presence of both metalloprotease and disintegrin domains, the ADAM proteins are related to a family of soluble snake venoms. In fact, the name ADAM for this family was chosen to reflect the potential function of these proteins as well as their dual origin in the fields of snakes and male fertility.

There are two ADAM6 proteins in mice, ADAM6a and ADAM6b (SEQ ID NO:1 and 2, respectively), encoded by two single-exon intronless genes that are both expressed, as detected by gene-specific RT-PCR [Choi, I. et al., Genomics (2004) 83: 636-646]. The *Adam6a* and *Adam6b* genes (SEQ ID NO:3 and 4, respectively) are located on chromosome 12 within the immunoglobulin heavy chain locus (*Igh*) between the *Ighv5-1p* and *Ighd1-1* gene segments but in opposite transcriptional orientation to the V_{H} gene segments. There is only a single human *ADAM6* gene, located within the *IGHV* locus on chromosome 14 between the *IGHV1-2* and *IGHVII-1-1P* gene segments. Human *ADAM6* has an in-**frame stop codon near the 5' end of the putative coding sequence and a** downstream frameshift and therefore is a pseudogene (Choi ibid.).

Macdonald et al. teach a method for very large, in situ, and precise genetic humanization of mice using large compound bacterial artificial chromosome-based targeting vectors introduced into mouse ES cells and describe use of this method to humanize segments of the mouse heavy and light chain Ig loci (Macdonald et al. 2014, PNAS, 111(14):5147-5152).

The mature ADAM6 protein is found on the sperm surface overlaying the acrosome and the posterior regions of the sperm head. ADAM6 was found to form a complex with ADAM2 and ADAM3 in testis, although the association with ADAM3 is thought to be indirect since this protein is localized on the anterior, rather than posterior region of the sperm head. Male mice with a deletion of *Adam2* or *Adam3* genes are infertile; *Adam2* knockout sperm show impaired migration from the uterus to the oviduct and both *Adam2* and *Adam3* knockout sperm are defective in binding to the zona pellucida. ADAM6 protein was almost totally absent in *Adam2* and *Adam3* knockout sperm, suggesting that ADAM2 and ADAM3 are required for the proper localization of ADAM6 protein on the sperm head.

The requirement for *Adam6* genes for full male fertility was discovered serendipitously during the generation of mice in which the endogenous mouse *Adam6* genes, which are located on chromosome 12 within the *Igh* locus, were deleted along with the mouse *Ighv, Ighd* and *Ighj* gene segments. Deleted mouse *Igh* gene segments were replaced with their human counterparts, thereby replacing the functional mouse *Adam6a* and *Adam6b* genes by the human ADAM6 pseudogene, with the consequence that such mice suffered from male infertility.

According to US8697940B2, mice with replaced immunoglobulin loci can comprise a disruption or deletion of the endogenous mouse ADAM6 locus, which is normally found **between the 3'**-most V_{H} **gene segment and the 5'**-most D gene segment at the mouse immunoglobulin heavy chain locus. Disruption in this region can lead to reduction or **elimination of functionality of the endogenous mouse ADAM6 locus. If the 3'**-most V_{H} gene segments of the human heavy chain repertoire are used in a replacement, an intergenic region containing the human *ADAM6* pseudogene is present between these V_{H} gene segments, i.e., between human *IGHV1-2* and *IGHVII-1-1P.* However, male mice that comprise this human intergenic sequence exhibit a severe reduction in fertility.

US8697940B2 (WO2012/141798A1) discloses genetically modified mice that comprise an ectopic mouse, rodent, or other *Adam6* gene (or ortholog or homolog or fragment) functional in a mouse, and one or more human immunoglobulin variable and/or constant region gene segments. A humanized heavy chain locus containing an ectopically placed genomic sequence comprising both mouse *Adam6a* and *Adam6b* nucleotide sequences was necessary to produce an ADAM6 rescue mouse with male fertility.

The publicly available and published literature indicates that both *Adam6a* and *Adam6b* genes are required for full male fertility. This issue was directly tested by Lee, et al. [Lee, E-C, Nature Biotech. (2014) 32:356-363], who made null mutations of *Adam6a* alone, resulting in a mouse with only *Adam6b* intact, or of both *Adam6a* and *Adam6b,* resulting in a complete ADAM6 deficiency. Males homozygous for either mutation had dramatically reduced fertility. Most of the males were completely infertile. The authors conclude that mouse variable regions should be inactivated by inversion rather than deletion to retain the *Adam6* genes in the mouse genome.

WO2017/201476A1 discloses transgenic mice, which lack a functional endogenous *Adam6* gene and comprise a functional ectopic nucleic acid sequence to complement the loss of the endogenous *Adam6* gene. If the mouse has a loss of a certain endogenous *Adam6* gene, it requires the same functional ectopic nucleic sequence to complement the loss. It follows from WO2017/201476A1 that if there is a lack of the two endogenous *Adam6* genes, namely the endogenous *Adam6a* and *Adam6b* genes, two ectopic functional *Adam6* genes are required to complement the loss.

WO2013/079953A1 discloses transgenic mice wherein expression of endogenous ADAM6 has been inactivated and at least one ADAM6a-encoding nucleotide sequences and at least one ADAM6b-encoding nucleotide sequence are inserted into the genome of the mice to rescue fertility of the mice.

There is a need to provide transgenic mice capable of producing monoclonal antibodies comprising human V_{H} genes with retained fertility.

### SUMMARY OF THE INVENTION

This summary is provided to introduce a selection of concepts in a simplified form that are further described below in the detailed description. This summary is not intended to identify key or essential features of the claimed subject matter, nor is it intended to be used to limit the scope of the claimed subject matter. Other features, details, utilities, and advantages of the claimed subject matter will be apparent from the following written detailed description, including those aspects illustrated in the accompanying drawings and defined in the appended claims.

It is the objective of the present invention to provide transgenic mice which comprise human V_{H} genes and which are fertile despite comprising a modification in their genome reducing expression of endogenous *Adam6a* and *Adam6b* genes.

The object is solved by the subject of the present claims and as further described herein.

The invention is defined as described in the claims. All other embodiments described herein are not part of the invention. According to the invention there is provided a transgenic mouse that is engineered by a deletion of endogenous *Adam6a* and *Adam6b* genes in a male mouse, wherein the mouse is homozygous for the deletion, and comprises in its genome only one exogenous *Adam6* transgene, and expresses an ADAM6 protein comprising at least 90% sequence identity to SEQ ID NO:2, which *Adam6* transgene is functional in a male mouse, wherein the mouse comprises an immunoglobulin heavy chain variable region (IHVR) locus comprising human immunoglobulin gene segment (hIGS) coding sequences and murine expression control sequences in operable linkage to control expression of the hIGS coding sequences, and wherein the exogenous Adam6 transgene is embedded in said IHVR locus, wherein said hIGS coding sequences and said murine expression control sequences are comprised in a heterologous expression cassette further comprising said *Adam6* transgene, wherein said heterologous expression cassette is integrated within the mouse genome and replaces endogenous immunoglobulin gene segment coding sequences, or replaces an immunoglobulin gene segment of the endogenous IHVR locus, and wherein said *Adam6* transgene is *Adam6b*.

Specifically, herein provided is a transgenic mouse which is engineered by one or more genetic modifications that reduce expression of endogenous *Adam6a* and *Adam6b* genes in a male mouse compared to a male mouse without said one or more genetic modifications, which mouse comprises in its genome only one exogenous *Adam6* transgene, and expresses an ADAM6 protein comprising at least 90% sequence identity to SEQ ID NO:2, which *Adam6* transgene is functional in a male mouse.

Specifically, the transgenic mouse described herein comprises in the genome of a cell only one exogenous *Adam6* transgene.

Specifically, the ADAM6 protein expressed by said *Adam6* transgene is functional in a male mouse.

Specifically, the invention provides for a strain of the transgenic mouse described herein.

Specifically, the *Adam6* transgene is *Adam6b*.

SEQ ID NO:1 identifies murine ADAM6a protein; and SEQ ID NO:2 identifies murine ADAM6b protein.

Specifically, the exogenous *Adam6* transgene expresses an ADAM6b protein which is 100% identical to mouse ADAM6b, respectively. Alternatively, the exogenous *Adam6* transgene expresses a functional variant of ADAM6b.

According to a specific example, the exogenous *Adam6* transgene is *Adam6b* (SEQ ID NO:4) expressing murine ADAM6b (SEQ ID NO:2).

Specifically, the exogenous *Adam6* transgene expresses an ADAM6 protein comprising at least any one of 90, 91, 92, 93, 94, 95, 96, 97, 98, 99 or 100% sequence identity to SEQ ID NO:2.

According to a specific embodiment, the ADAM6 protein expressed by the *Adam6* transgene is at least any one of 95, 96, 97, 98, or 99% identical, or is 100% identical to any one of SEQ ID NO:2.

Specifically, the *Adam6* transgene is functionally active in a male mouse, such that it expresses a functional ADAM6 protein or a function variant thereof with a certain sequence identity. A functional variant of an ADAM6 protein is characterized in that it restores fertility in the transgenic male mouse described herein such that the mouse is capable of reproduction. Specifically, the transgenic mouse is able to produce offspring in litter sizes comparable to those of wild type mice.

Fertility is typically measured as the number of viable pups per litter at birth. A mouse is considered fertile if at least one pup is viable at birth. In a wild-type fertile mouse, specifically a C57BL/6 mouse, fertility results in an average of five to six pups per female and the average pregnancy rate is ~85%.

Specifically, the ADAM6 protein expressed by the *Adam6* transgene described herein is functional with regard to the fertility of a male mouse. A substantial reduction in fertility is, e.g., a reduction in fertility (e.g., breeding frequency, pups per litter, litters per year, etc.) of about 50%, 60%, 70%, 80%, 90%, or 95% or more. The exogenous *Adam6* transgene described herein rescues the reduction in fertility in whole or in substantial part. A rescue of fertility is, e.g., a restoration of fertility such that the non-human animal exhibits a fertility that is at least 70%, 80%, or 90% or more as compared with, e.g., a wild-type mouse or a mouse comprising no modification reducing expression of the endogenous *Adam6a* and *Adam6b* genes.

It surprisingly has turned out that mice comprising only one of *Adam6a* or *Adam6b* as an exogenous *Adam6* transgene did not suffer from reduction in fertility. The transgenic mouse provided herein is fertile, despite comprising one or more genetic modifications reducing or eliminating expression of endogenous *Adam6* genes.

In particular, fertility has proven if the *Adam6* transgene was embedded in an immunoglobulin heavy chain gene segment that comprises mouse intergenic regions. Mouse expression control sequences could be used to effectively express a functional ADAM6 protein from only one *Adam6* transgene comprised at the immunoglobulin heavy chain variable region locus.

Specifically, the mouse is homozygous or heterozygous with respect to said exogenous *Adam6* transgene.

Specifically, the mouse is homozygous or heterozygous for the genetic modification, deleting or inactivating endogenous *Adam6a* and *Adam6b.*

The genetic modification reducing expression of endogenous *Adam6a* and *Adam6b* may be the consequence of replacing immunoglobulin heavy chain variable regions within the mouse genome.

Alternatively, such genetic modification may be on purpose e.g., to replace the endogenous *Adam6a* and *Adam6b* genes by the exogenous *Adam6* transgene as described herein, which is incorporated within the mouse genome at a predetermined position.

Specifically, reduction of expression of endogenous *Adam6a* and *Adam6b* refers to complete reduction or elimination of expression.

Specifically, said one or more genetic modifications comprises a disruption, substitution, deletion or knockout of (i) one or more endogenous polynucleotides, or a part thereof; or (ii) an expression control sequence, preferably the expression control sequence is selected from the group consisting of a promoter, transcriptional start and stop sequences, enhancer and activator sequences, a ribosome binding site, or translational start and stop sequences.

A genetic modification may comprise partial deletion or full deletion, also referred to as "knock-out", of the *Adam6a* and *Adam6b* genes or inactivating mutations, also referred to as "loss-of-function" mutations including e.g. point mutations or frameshift mutations. An inactivating mutation may comprise a sequence modification to alter one or more contiguous amino acids, e.g. 1, 2, or 3 amino acids, which are substituted, inserted or deleted at one position in an amino acid sequence expressed by the respective gene. An inactivating mutation may also refer to areas of sequences, in particular changing contiguous or non-contiguous amino acid sequences.

Specifically, the endogenous *Adam6a* and *Adam6b* genes are knocked out by said one or more genetic modifications.

The genetic modification may comprise a replacement, wherein a nucleic acid element is placed into the mouse genome by introducing and incorporating a heterologous nucleic acid construct at the locus of the genomic sequence. The DNA sequence so placed may include one or more regulatory sequences that are part of source DNA used to obtain the sequence so placed (e.g., promoters, enhancers, 5'- or 3'-untranslated regions, appropriate recombination signal sequences, etc.). For example, in various embodiments, the replacement is a substitution of an endogenous sequence for a heterologous sequence that results in the production of a gene product from the DNA sequence so placed (comprising the heterologous sequence), but not expression of the endogenous sequence. In a specific embodiment, an immunoglobulin gene segment is replaced with a corresponding chimeric or human immunoglobulin gene segment or locus. In another specific embodiment, endogenous immunoglobulin gene segment coding sequences are replaced with corresponding human immunoglobulin gene segment coding sequences, such as an ortholog of, a homolog of endogenous sequences, or those that are at least 90% or 95%, or 100% identical to the endogenous sequences that are replaced. In yet another specific embodiment, endogenous immunoglobulin gene segment coding sequences are replaced with corresponding human immunoglobulin gene segment coding sequences embedded in mouse non-coding and regulatory sequences.

The genome of the mouse described herein comprises human immunoglobulin regions. Specifically, the human immunoglobulin regions are human immunoglobulin variable regions, such as V_{H} and optionally V_{L} coding sequences.

Transgenic mice as described herein, in particular those that comprise a replacement at the endogenous immunoglobulin heavy chain locus with heterologous or recombinant (e.g., from another species) immunoglobulin sequences, can be made in conjunction with replacements at endogenous immunoglobulin light chain loci or in conjunction with immunoglobulin light chain transgenes (e.g., chimeric immunoglobulin light chain transgenes, or fully human or fully mouse immunoglobulin light chain transgenes, etc.). The species from which the heterologous immunoglobulin heavy chain sequences are derived can vary widely; as with immunoglobulin light chain sequences employed in immunoglobulin light chain sequence replacements or immunoglobulin light chain transgenes.

Immunoglobulin variable region nucleic acid sequences, e.g., V, D, and/or J segments, are specifically obtained from a human or a non-human animal. Various non-human animals can be used as sources of variable domains or variable region gene segments and respective immunoglobulin coding sequences. Non-human animals suitable for providing V, D, and/or J segments or respective immunoglobulin coding sequences include, for example non-human primates (e.g., chimpanzees, orangutans, gorillas, marmosets, rhesus monkeys, baboons) or rodents including rats, mice, and hamsters. Further non-human animals suitable as a source for providing immunoglobulin variable region nucleic acid sequences include, for example, Canidae, such as e.g. dogs and wolves; Felidae, such as e.g. cats and tigers; Bovidae such as e.g. sheep, goats and cows; Fish, Agnatha (e.g., lamprey and hagfish) and Gnathostomata (e.g., cartilaginous and bony fish); Equidae such as e.g., horses; and Phasianidae, such as e.g., chicken.

Numerous methods have been developed for replacing endogenous mouse immunoglobulin regions with human immunoglobulin sequences to create partly- or fully-human antibodies, specifically for drug discovery purposes. Examples of such mice include those described in, for example, U.S. Pat Nos. 7,145,056; 7,064,244; 7,041,871; 6,673,986; 6,596,541; 6,570,061; 6,162,963; 6,130,364; 6,091,001; 6,023,010; 5,593,598; 5,877,397; 5,874,299; 5,814,318; 5,789,650; 5,661,016; 5,612,205; and 5,591,669.

The mouse may comprise human, chimeric and/or rodent (e.g., mouse or rat) constant region genes or sequences. Yet, according to a specific aspect, the mouse may comprise a deletion of an immunoglobulin heavy chain constant region gene sequence.

The mouse may comprise a human, humanized, or chimeric immunoglobulin light chain genes or sequences. Yet, according to a specific aspect, the mouse may comprise a genetic modification inactivating, disabling or deleting the endogenous immunoglobulin light chain locus or parts thereof.

In one aspect, the transgenic mouse described herein comprises
a) a replacement of one or more, in particular all mouse V_{H} gene segments or V_{H} gene segment coding sequences with one or more human V_{H} gene segment coding sequence(s), respectively; and/or;
b) a replacement of one or more, in particular all mouse D_{H} gene segments or D_{H} gene segment coding sequences with one or more human D_{H} gene segment coding sequence(s), respectively; and/or
c) a replacement of one or more, in particular all mouse J_{H} gene segments or J_{H} gene segment coding sequences with one or more human J_{H} gene segment coding sequence(s), respectively.

In a specific aspect, the endogenous *Adam6a* and *Adam6b* genes of the transgenic mouse provided herein are knocked-out by deleting the endogenous mouse *Ighv* locus and replacement of the endogenous locus with exogenous *IGHV, IGHD* and *IGHJ* gene segment coding sequences, specifically human *IGHV, IGHD* and *IGHJ* gene segment coding sequences.

According to a specific embodiment, the endogenous *Adam6a* and *Adam6b* genes of the transgenic mouse provided herein are deleted by replacing the endogenous mouse *Ighv* locus and with a chimeric *IGHV* locus comprising human immunoglobulin gene segment (hIGS) coding sequences and murine expression control sequences in operable linkage to control expression of the hIGS coding sequences. Specifically, the chimeric *IGHV* locus comprises the exogenous *Adam6* transgene described herein.

According to a further specific embodiment, the expression of endogenous *Adam6a* and *Adam6b* genes of the transgenic mouse provided herein is reduced by a genetic modification and in a second modification endogenous immunoglobulin gene segment coding sequences or endogenous immunoglobulin heavy chain gene segments are replaced by a heterologous expression cassette as described herein. Specifically, the heterologous expression cassette comprises the exogenous *Adam6* transgene described herein and heterologous immunoglobulin gene segment coding sequences or heterologous immunoglobulin heavy chain gene segments as described herein.

The transgenic mouse provided herein comprising the exogenous *Adam6* transgene is useful where modifications disrupt the function of endogenous mouse ADAM6a and ADAM6b. The probability of disrupting endogenous mouse ADAM6 function is high when making modifications to mouse immunoglobulin loci, in particular when modifying mouse immunoglobulin heavy chain variable regions and surrounding sequences. The position of the intergenic sequence in mice that encodes ADAM6a and ADAM6b renders the intergenic sequence susceptible to modification when modifying an endogenous mouse heavy chain locus. When V_{H} gene segments are deleted or replaced, or when D_{H} gene segments are deleted or replaced, there is a high probability that expression of endogenous *Adam6a* and *Adam6b* genes is reduced in the male mouse. Therefore, the transgenic mouse described herein provides particular benefit when making mice with immunoglobulin heavy chain loci that are deleted in whole or in part, are humanized in whole or in part, or are replaced in whole or in part.

The mouse described herein comprises an immunoglobulin heavy chain variable region (IHVR) locus comprising human immunoglobulin gene segment (hIGS) coding sequences and murine expression control sequences in operable linkage to control expression of the hIGS coding sequences. Specifically, said IHVR locus is on chromosome 12.

Said hIGS coding sequences and said murine expression control sequences are comprised in a heterologous expression cassette further comprising said *Adam6* transgene, wherein said heterologous expression cassette is integrated within the mouse genome and replaces endogenous immunoglobulin gene segment coding sequences, or replaces an immunoglobulin gene segment of the endogenous IHVR locus.

Specifically, the mouse described herein comprises chimeric immunoglobulin segments such as described in US 2013/0219535, specifically, human IGH (V_{H}, D_{H}, J_{H}), IGK (VK, JK), and IGL (V_{λ}, and J_{λ}) coding sequences embedded in the corresponding mouse non-coding and regulatory sequences. An immunoglobulin gene segment comprising hIGS coding sequences and murine expression control sequences are herein also referred to as "chimeric immunoglobulin gene segments".

Specifically, such transgenic mice have a genome comprising an introduced exogenous immunoglobulin region, which is partly human, where the introduced region comprises human variable region coding sequences and murine non-coding regulatory sequences controlling expression of the human sequences, which are of the mouse origin or of the endogenous genome of a mouse and knocked into the mouse genome by introduction of chimeric immunoglobulin gene segments or a chimeric immunoglobulin gene locus. In particular, the murine sequences, specifically the murine non-coding and regulatory sequences, are based on the corresponding mouse endogenous sequences, i.e. of an identical sequence as those of the endogenous wild-type immunoglobulin locus.

Murine expression control sequences as described herein for the purpose of expressing human immunoglobulin gene sequences are specifically selected from the group consisting of a promoter, transcriptional start and stop sequences, enhancer and activator sequences, a ribosome binding site, or translational start and stop sequences. Specific examples of such expression control sequences are sequences flanking the coding sequence, which may include the promoter, 5' untranslated sequence, intron intervening the coding sequences of the leader peptide, recombination signal sequence(s) (RSS), and splice sites.

The *Adam6* transgene, described herein, that rescues fertility can be placed at any suitable position. In one embodiment, the nucleotide sequence can be introduced as a transgene that randomly integrates into the mouse genome. The exogenous *Adam6* transgene can, e.g., be integrated at the position of any one of the endogenous *Adam6a* and *Adam6b* genes, at a position that approximates its location in a wild-type mouse (e.g., in a modified endogenous mouse immunoglobulin locus upstream or downstream of its natural position), at a location identical to the endogenous position, or at a position different to the endogenous position.

Specifically, the *Adam6* transgene is comprised in a nucleic acid construct comprising at least one immunoglobulin gene segment coding sequence, in particular at least V_{H}, D_{H} and J_{H} gene segment coding sequences of an immunoglobulin heavy chain locus. Such construct is herein also referred to as "immunoglobulin gene locus".

Specifically, the *Adam6* transgene is comprised in a nucleic acid construct comprising at least one immunoglobulin gene segment of an immunoglobulin heavy chain locus.

Specifically, the *Adam6* transgene is comprised in such nucleic acid construct or immunoglobulin gene locus which is chimeric (herein referred to as "chimeric immunoglobulin gene locus"), in particular wherein such construct comprises human immunoglobulin coding sequences and murine regulatory sequences. Specifically, the *Adam6* transgene is comprised in such nucleic acid construct or immunoglobulin gene locus, and positioned between immunoglobulin heavy chain V_{H} and D_{H} gene segments, in particular positioned in the opposite transcriptional orientation.

The immunoglobulin gene locus comprising the *Adam6* transgene can be either exogenous and introduced into the mouse genome, or endogenous. An immunoglobulin gene locus comprising the *Adam6* transgene can be introduced into the mouse genome as an exogenous nucleic acid construct or element. Alternatively, the *Adam6* transgene is knocked into the immunoglobulin gene locus within the mouse genome.

Specifically, the exogenous Adam6 transgene is knocked in by incorporating an exogenous chimeric immunoglobulin gene locus comprising said exogenous *Adam6* transgene.

Specifically, the *Adam6* transgene is embedded within an IHVR locus.

Specifically, the *Adam6* transgene is knocked in at the mouse IHVR locus, thereby incorporating said *Adam6* transgene within the locus.

Specifically, the *Adam6* transgene is embedded between immunoglobulin heavy chain variable region (*IGHV*) coding genes and diversity (*IGHD*) coding genes.

Specifically, the *Adam6* transgene is embedded between the *IGHV* and the *IGHD* coding sequences, preferably in opposite transcriptional orientation to the *IGHV* gene segment coding sequences.

The *Adam6* transgene may be positioned between two V_{H} gene segments, between a V_{H} and a D_{H} gene segment or between a D_{H} and a J_{H} gene segment.

In a specific aspect, the exogenous *Adam6* transgene is located between V_{H} and D_{H} gene segments, which may be mouse, chimeric and/or human. Specifically, it is placed between the 3'-most V_{H} gene segment and the 5'-most D_{H} gene segment at the immunoglobulin heavy chain locus.

According to a specific aspect, the exogenous *Adam6* transgene is knocked in between the hIGS coding sequences.

According to another specific aspect, the exogenous *Adam6* transgene is knocked in between two different immunoglobulin gene segment coding sequences, in particular wherein each of the two different immunoglobulin gene segment coding sequences are chimeric, such that the *Adam6* transgene is embedded between the immunoglobulin variable region gene segment coding sequences.

Specifically, the exogenous *Adam6* transgene is operably linked to at least one murine expression control sequence, such as a promoter or enhancer. Specifically, expression of the *Adam6* transgene embedded in said hIGS is controlled by endogenous murine expression control sequences, or those which are of mouse immunoglobulin locus origin. The promoter and putative enhancers that regulate expression of *Adam6a*/ *b* genes in wild-type mice may be used. The Cdx1 and Ctcf binding sites identified by Choi, et al. as promoter and negative regulatory regions of the Adam2 gene (Mol. Biol. Rep. (2013) 40:787-796) are conserved in the ~400bp sequence upstream of the transcription start sites of both *Adam6a* and *Adam6b* genes. Moreover, two peaks at -51 and -92 of relatively high GC content (53% and 56%, respectively), which are typical of eukaryotic promoter regions, are also present upstream of both *Adam6a* and *Adam6b* genes.

Specifically, murine *Adam6* expression control sequences are used which are preferably endogenous in the mouse genome (in particular wild-type murine expression control sequences) or originating from such mouse genome. The upstream region described above can be tested and used to drive expression of the *Adam6* transgene if it is inserted in an ectopic location in the mouse genome.

Specifically, the mouse is heterozygous or homozygous for the exogenous *Adam6* transgene described herein. Specifically, there is only one exogenous *Adam6* transgene on one allele or in the genome of a cell of the transgenic mouse described herein. Specifically, there is one exogenous *Adam6* transgene on each allele of a chromosome of the genome of the transgenic mouse described herein. Specifically, the *Adam6* transgene is *Adam6b*, as further described herein.

Specifically, said exogenous *Adam6* transgene is comprised in a heterologous expression cassette.

Specifically, the heterologous expression cassette comprises the exogenous *Adam6* transgene in operable linkage with nucleotide sequences controlling expression of said *Adam6* transgene.

According to a specific embodiment, the heterologous expression cassette comprises the exogenous *Adam6* transgene, and the expression cassette is integrated into the mouse genome such that expression of the *Adam6* transgene is under the control of murine expression control sequences which are present at the IHVR locus (before or after expression cassette integration) or which are endogenous mouse expression control sequences, specifically endogenous murine Adam6 expression control sequences.

Specifically, the heterologous expression cassette not only comprises the exogenous *Adam6* transgene, but further comprises hIGS coding sequences. According to a specific embodiment the expression cassette further comprises murine expression control sequences in operably linkage to control expression of the exogenous *Adam6* transgene and murine expression control sequences in operably linkage to control expression of hIGS coding sequences. Specifically, such murine expression control sequences in operable linkage with *Adam6* and those in operable linkage with hIGS coding sequences are different.

Specifically, the hIGS coding sequences are of immunoglobulin heavy chain variable region (*IGHV*), diversity (*IGHD*) and joining (*IGHJ*) gene segments.

Specifically, human *IGHV, IGHD,* and *IGHJ* genes are comprised at the IHVR locus, in particular in respective immunoglobulin gene segments, which are preferably chimeric immunoglobulin gene segments. Specifically, each of the immunoglobulin gene segments are chimeric immunoglobulin gene segments.

According to a specific aspect, the mouse comprises immunoglobulin gene segments that upon VDJ_{H}, VJ_{κ} or VJ_{λ} rearrangement encode immunoglobulin sequences of one or more antibody variable domains. Specifically, the mouse expresses a repertoire of human or humanized antibody variable domains.

Specifically, at least one or more (or each) of the immunoglobulin heavy chain variable region gene segments are chimeric immunoglobulin gene segments.

Specifically, at least one or more (or each) of the immunoglobulin light chain variable region gene segments, which may be κ or λ, are chimeric immunoglobulin gene segments.

Said heterologous expression cassette replaces endogenous immunoglobulin gene segment coding sequences, or replaces an immunoglobulin gene segment of the endogenous IHVR locus.

Further provided herein is a recombinant expression cassette comprising human immunoglobulin gene segment (hIGS) coding sequences and murine expression control sequences in operable linkage to control expression of the hIGS coding sequences, and only one *Adam6* transgene capable of expressing an ADAM6 protein comprising at least 90% sequence identity to SEQ ID NO:2, which *Adam6* transgene is functional in a male mouse, wherein said *Adam6* transgene is *Adam6b*. Specifically, said *Adam6* transgene expresses an ADAM6 protein comprising at least any one of 90, 91, 92, 93, 94, 95, 96, 97, 98, 99 or 100% sequence identity to SEQ ID NO:2. According to a specific embodiment, the ADAM6 protein expressed by said *Adam6* transgene is at least any one of 95, 96, 97, 98, or 99% identical, or is 100% identical to SEQ ID NO:2.

Such recombinant expression cassette can, for example, be provided in the form of a recombinase mediated cassette exchange (RMCE) vector.

Specifically, the recombinant expression cassette is used to engineer the transgenic mouse as further described herein. For this purpose, the *Adam6* transgene comprised in the recombinant expression cassette represents the exogenous Adam6 transgene when incorporated in the mouse genome.

Specifically, the recombinant expression cassette provided herein comprises said murine expression control sequences as intergenic sequences embedded in a gene segment comprising hIGS coding sequences.

Specifically, the hIGS coding sequences comprised in the recombinant expression cassette provided herein comprise or consist of sequences encoding human heavy chain variable sequences, in particular those which upon VDJ rearrangement encode immunoglobulin sequences of one or more antibody variable domains, or which are already rearranged.

According to a specific embodiment, the recombinant expression cassette provided herein comprises:
a) human V_{H} gene segment coding sequences including heavy chain variable region gene segment (*IGHV*) coding sequences, diversity gene segment (*IGHD*) coding sequences, and joining gene segment (*IGHJ*) coding sequences; and
b) said *Adam6* transgene embedded between the *IGHV* and the *IGHD* coding sequences, preferably in opposite transcriptional orientation to the V_{H} gene segment coding sequences.

Further provided herein is an isolated chromosomal region comprising an immunoglobulin heavy chain variable region locus comprising the expression cassette described herein, which is functional to express the one or more antibody heavy chain variable domains in a mouse.

Further provided herein is a method for producing the mouse described herein, comprising:
a) providing an embryonic mouse stem cell;
b) introducing one or more genetic modifications reducing expression of endogenous *Adam6a* and *Adam6b* genes, preferably by knocking out the *Adam6a* and *Adam6b* genes;
c) providing one or more vectors comprising at least one expression cassette containing gene segments that upon VDJ_{H} rearrangement encode immunoglobulin sequences;
d) introducing said one or more vectors into the cell;
e) incorporating said gene segments into the genome of the cell, and selecting a transgenic cell wherein said gene segments have been integrated into the cellular genome of said transgenic cell at a target site at the endogenous immunoglobulin heavy chain gene locus; and
f) utilizing said transgenic cell to create a transgenic mouse comprising said transgenic cell;
wherein at least one of said vectors comprises the expression cassette described herein.

Specifically, the method of producing the mouse described herein comprises:
a) providing an embryonic mouse stem cell;
b) providing one or more vectors comprising at least one expression cassette containing gene segments that upon VDJ rearrangement encode immunoglobulin sequences;
c) introducing said one or more vectors into the cell;
d) incorporating said gene segments into the genome of the cell, and selecting a transgenic cell wherein said gene segments have been integrated into the cellular genome of said transgenic cell at a target site at the endogenous immunoglobulin heavy chain gene locus, thereby deleting endogenous *Adam6a* and *Adam6b* genes; and
e) utilizing said transgenic cell to create a transgenic mouse comprising said transgenic cell;
wherein at least one of said vectors comprises the expression cassette described herein.

According to a specific embodiment of the method provided herein, expression of endogenous *Adam6a* and *Adam6b* genes is reduced by deleting all or a part of the endogenous immunoglobulin heavy chain variable locus.

According to a further specific embodiment, expression of endogenous *Adam6a* and *Adam6b* genes is reduced by replacing endogenous immunoglobulin gene segment coding sequences or an endogenous IHVR locus with the heterologous expression cassette described herein.

Specifically, a marker is used to indicate the successful integration of said gene segments into the cellular genome. Specifically, the marker is a selectable marker, which is capable of expression in a host that allows for ease of selection of those hosts containing an introduced nucleic acid or vector.

Examples of selectable markers include, e.g., proteins that confer resistance to antimicrobial agents (*e.g*., puromycin, hygromycin, bleomycin, phleomycin D1, or neomycin), proteins that confer a metabolic advantage, such as a nutritional advantage on the host cell, as well as proteins that confer a functional or phenotypic advantage (*e.g*., cell division) on a cell.

Specifically, said expression cassette comprising said gene segments and/or said exogenous *Adam6* transgene is integrated into the cellular genome of said mouse cell at a target site, by any method of targeted recombination, *e.g.,* by homologous recombination or by site-specific recombination techniques. Specifically, the CRISPR/Cas9 genome editing system (He et al., Nucleic Acids Research 2016, 44:e85) or the CRISPR-associated transpose-mediated DNA insertion system (Strecker, et al., Science 2019, 365:448; Klompe, et al, Nature, 2019, 571:219) may be used for targeted recombination.

Further provided herein is the use of the mouse described herein for the production of antibodies.

Further provided herein is the use of the mouse described herein for the generation of a chimeric or humanized antibody comprising human variable regions and non-human animal *e.g*., rodent such as mouse or rat, constant regions, or a fully human antibody.

Non-human animals may be used as sources of constant region sequences to be used in connection with variable sequences, for example, rodent constant sequences can be used in transgenes operably linked to human, chimeric or non-human variable sequences (e.g., human variable sequences operably linked to, e.g., rodent, e.g., mouse or rat or hamster, constant sequences). Thus, in various embodiments, human or chimeric V, D, and J gene segment coding sequences can be operably linked to rodent (e.g., mouse or rat or hamster) constant region gene sequences. In some embodiments, the human or chimeric V, D, and J gene segment coding sequences (or one or more rearranged VDJ genes) are operably linked or fused to a mouse, rat, or hamster constant region gene sequence in, e.g., a transgene integrated at an IHVR locus.

According to a specific embodiment, the transgenic mouse provided herein is immunized with an antigen such that an immune response is elicited against that antigen resulting in the generation of affinity-matured specific monoclonal or polyclonal antibodies. An antigen can be administered to the mouse in any convenient manner to trigger an immune response, with or without an adjuvant, and can be administered in accordance with a predetermined immunization schedule.

After immunization, serum or milk from immunized mice can be fractionated for the purification of pharmaceutical grade polyclonal antibodies specific for the antigen. A concentrated, purified immunoglobulin fraction may be obtained by chromatography (affinity, ionic exchange, gel filtration, etc.), selective precipitation with salts such as ammonium sulfate, organic solvents such as ethanol, or polymers such as polyethylene glycol.

For making a monoclonal antibody, antibody-producing cells, e.g., spleen and/or lymph node cells, may be isolated from the immunized transgenic mouse and used either in cell fusion with transformed cell lines for the production of hybridomas, or cDNAs encoding antibodies are cloned by standard molecular biology techniques and expressed in transfected cells. The procedures for making monoclonal antibodies are well established in the art.

Specifically, the method further comprises the step of isolating nucleic acid sequences from the immunized mouse described herein for the production of specific antibodies, or fragments thereof, in particular antigen-binding fragments, in a cell culture. Such antibodies or antigen-binding fragments thereof can be hyperimmune antibodies.

Further described herein, but not part of the invention, is an isolated mouse cell whose genome comprises:
a) a knockout of the endogenous *Adam6a* and *Adam6b* genes*,* and only one exogenous *Adam6* transgene expressing an ADAM6 protein comprising at least 90% sequence identity to SEQ ID NO:1 or SEQ ID NO:2, which exogenous *Adam6* transgene is functional in a male mouse; and
b) an immunoglobulin heavy chain variable region (IHVR) locus comprising human immunoglobulin gene segment (hIGS) coding sequences and murine expression control sequences in operable linkage to control expression of the hIGS coding sequences, preferably wherein said IHVR locus is on chromosome 12;
wherein the *Adam6* transgene is present at the IHVR locus.

Specifically, the hIGS coding sequences are operably linked to one or more mouse immunoglobulin constant region coding sequence(s) or gene(s) encoding one or more mouse immunoglobulin constant region domains.

Specifically, the isolated mouse cell described herein is a cell of a B-cell lineage or of a plasma cell lineage, specifically a mature B cell, a memory B cell, a plasmablast, or a plasma cell. Specific embodiments refer to primary B cells, immortalized B cells, or hybridomas derived from the transgenic mouse described herein.

Specifically, the genome of the isolated mouse cell comprises an exogenous *Adam6* transgene expressing an ADAM6 protein comprising at least any one of 90, 91, 92, 93, 94, 95, 96, 97, 98, 99 or 100% sequence identity to any one or both of SEQ ID NO:1 or SEQ ID NO:2. According to a specific embodiment, the ADAM6 protein expressed by said *Adam6* transgene is at least any one of 95, 96, 97, 98, or 99% identical, or is 100% identical to SEQ ID NO:2.

Further described herein, but not part of the invention, is a method for modifying an immunoglobulin heavy chain variable region (IHVR) locus of a mouse, comprising making a first modification of the IHVR locus that results in the reduction or elimination of expression of endogenous *Adam6a* and *Adam6b* genes in a male mouse, and making a second modification of the IHVR locus comprising inserting only one exogenous *Adam6* transgene expressing an ADAM6 protein comprising at least 90% sequence identity to SEQ ID NO:1 or SEQ ID NO:2, which *Adam6* transgene is functional in a male mouse, preferably wherein said first and second modifications are made simultaneously. Specifically, the *Adam6* transgene expresses an ADAM6 protein comprising at least 90, 91, 92, 93, 94, 95, 96, 97, 98, 99 or 100% sequence identity to SEQ ID NO:1 or SEQ ID NO:2. According to a specific embodiment, the ADAM6 protein expressed by said *Adam6* transgene is at least any one of 95, 96, 97, 98, or 99% identical, or is 100% identical to SEQ ID NO:2.

Specifically, the method for modifying an immunoglobulin heavy chain variable region (IHVR) locus of a mouse comprises making a first modification of the IHVR locus that deletes endogenous *Adam6a* and *Adam6b* genes in a male mouse, wherein the mouse is homozygous for the deletion, and making a second modification of the IHVR locus comprising inserting only one exogenous *Adam6* transgene expressing an ADAM6 protein comprising at least 90% sequence identity to SEQ ID NO:1 or SEQ ID NO:2, which *Adam6* transgene is functional in a male mouse, wherein said first and second modifications are made simultaneously.

Some further exemplary embodiments provide transgenic mice as described herein, further comprising changes to the immunoglobulin heavy chain gene to allow for production of bispecific antibodies such as described in WO2017035252A1.

Other embodiments include a part of or a whole immunoglobulin protein transcribed from the immunoglobulin heavy chain genes from the engineered portion of the transgenic mouse described herein; and part of or whole engineered immunoglobulin proteins derived from cells of the transgenic mouse described herein.

These and other aspects, objects and features of the invention are described in more detail below.

### FIGURES

**Figure 1****:** Illustrates the domain structure and proteolytic processing of the ADAM6 precursor protein to remove the prodomain.
**Figure 2****:** Diagram, not to scale, of the mouse *Adam6a* and *Adam6b* genes in the embedded in the TRN0010 humanized mouse *Igh* locus on chromosome 12. The transcriptional orientation of the *Adam6a, Adam6b* and *IGHV* gene segment coding sequences is indicated by the arrows. *IGHV,* heavy chain variable region gene segment coding sequences; *IGHD,* diversity gene segment coding sequences; *IGHJ,* joining gene segment coding sequences.

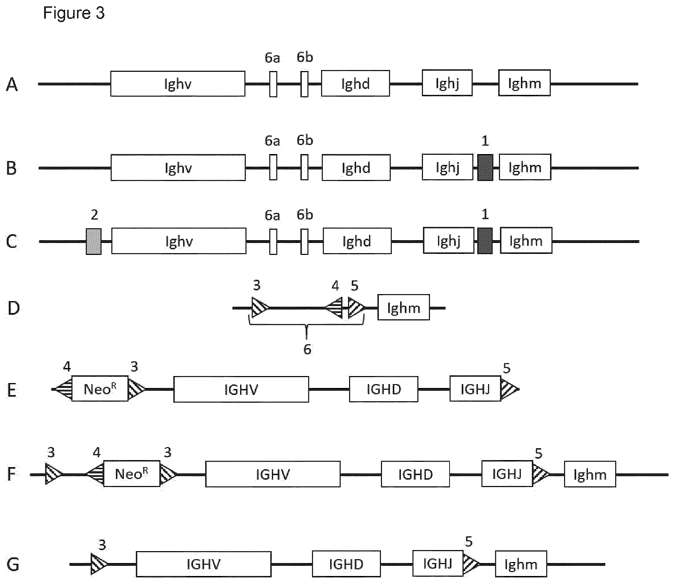

**Figure 3****:** Schematic of the methods used to generate the TRN0001 mouse by recombinase mediated cassette exchange (RMCE). (A) Schematic of the endogenous mouse IHVR allele in ES cells showing Ighv (V_{H} gene segments), endogenous mouse Adama6a (6a) and Adam6b (6b) genes, Ighd (D_{H} gene segments), Ighj (J_{H} gene segments), and Ighm (IgM constant region gene). (B) The same allele after insertion of the construct for the first part of the recombination docking site (1) downstream of the most proximal (relative to Ighm) Ighj gene segment by homologous recombination. (C) The same allele as in (B) after insertion of the second part of the recombination docking site docking construct (2) upstream of the most distal Ighv gene segment by homologous recombination. (SEQ ID NO:5 shows the sequences flanking the recombination sites (200 bp) for TRN0001.) (D) The configuration of the allele after *in vitro* deletion of the endogenous IHVR locus by Cre recombinase (3, FRT site; 4, Iox5171 site; 5, loxP site). These sites are contained in the docking constructs (1, 2 in Fig. 3) and are described in detail in US 2013/0219535. The Cre-mediated deletion creates the recombination docking site (6) to be used for RMCE. (E) Schematic of the synthetic RMCE vector containing human IGHV, IGHD and IGHJ coding sequences embedded in mouse flanking and regulatory sequences. A neomycin resistance gene (Neo^{R}**) is contained at the 5' end of the vector for positive selection purposes. (F) The** targeted humanized IHVR allele in ES cells after successful RMCE. (G) The final allele configuration after removal of the Neo^{R} gene by *in vivo* Flp recombination. Note the absence of any Adam6 genes.

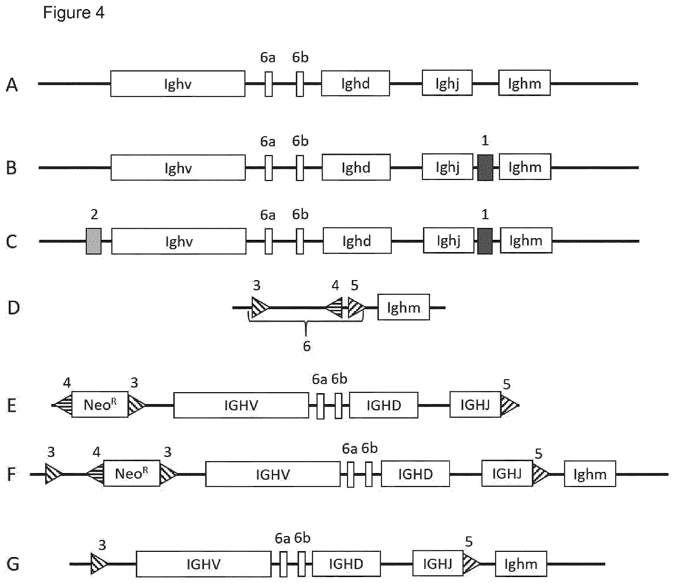

**Figure 4****:** Schematic of the methods used to generate the TRN0010 mouse by recombinase mediated cassette exchange (RMCE). (A) Schematic of the endogenous mouse IHVR allele in ES cells showing Ighv (V_{H} gene segments), endogenous moue Adama6a (6a) and Adam6b (6b) genes, Ighd (D_{H} gene segments), Ighj (J_{H} gene segments), and Ighm (IgM constant region gene). (B) The same allele after insertion of the first part of the recombination docking site construct (1) downstream of the most proximal (relative to Ighm) Ighj gene segment by homologous recombination. (C) The same allele as in (B) after insertion of the second part of the recombination docking site docking construct (2) upstream of the most distal Ighv gene segment by homologous recombination. (SEQ ID NO:5 shows the sequences flanking the recombination sites (200 bp) for TRN0010, which are the same sequences as for TRN0001.) (D) The configuration of the allele after *in vitro* deletion of the endogenous IHVR locus by Cre recombinase (3, FRT site; 4, Iox5171 site; 5, IoxP site). These sites are contained in the docking constructs (1, 2 in Fig. 4) and are described in detail in US 2013/0219535. The Cre-mediated deletion creates the recombination docking site (6) to be used for RMCE. (E) Schematic of the synthetic RMCE vector containing human IGHV, mouse Adam6a (6a), mouse Adam6b (6b) genes, IGHD, and IGHJ coding sequences embedded in mouse flanking and regulatory sequences. A neomycin resistance gene (Neo^{R}**) is contained at the 5' end of the vector for positive** selection purposes. (F) The targeted humanized IHVR allele in ES cells after successful RMCE. (G) The final allele configuration after removal of the Neo^{R} gene by *in vivo* Flp recombination. Note the presence of transgenic mouse Adam6 genes, both Adam6a and Adam6b. The sequence of the intergenic region between IGHV and IGHD that contains in Adam6a and Adam6b genes is shown in SEQ ID NO:6

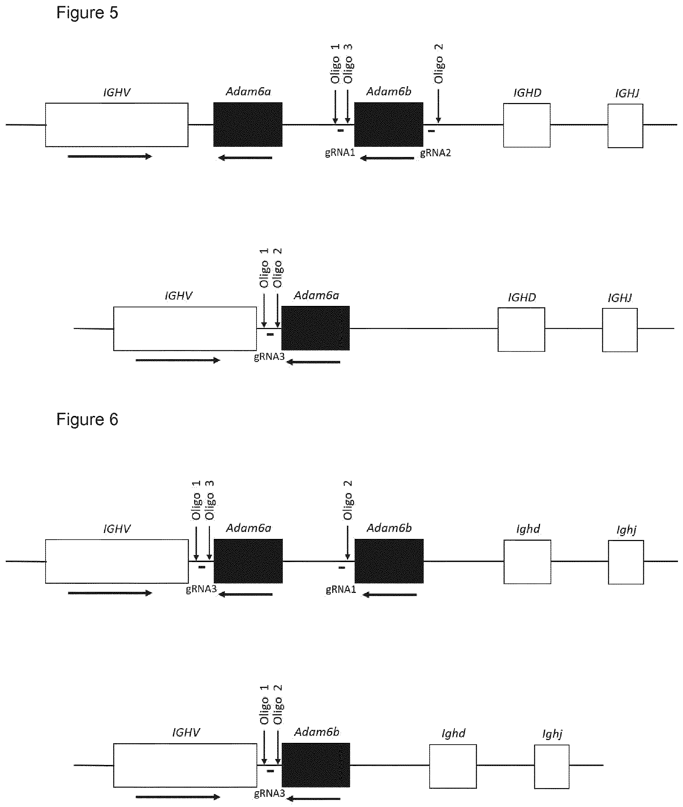

**Figure 5****:** Shows: (1, upper) The location of the proximal guide RNA binding site (gRNA1, SEQ ID NO:7) and distal guide RNA binding site (gRNA2, SEQ ID NO:8) used for the CRISPR/Cas9 mediated knockout of the *Adam6b* gene in TRN0010 (See Fig. 4G for a schematic showing the partly human TRN0010 heavy chain locus containing *Adam6* genes) **homozygous zygotes. [The last 3 nucleotides at the 3' end of these sequences correspond** to the Protospacer Adjacent Motif (PAM) required for Cas9 function.] (2, lower) The structure of the locus after deletion of *Adam6b* and the location of the binding sites for the oligonucleotides used for confirmation of the *Adam6b* gene knockout in founder mice by genomic PCR. Oligo 1, SEQ ID NO:9, Oligo 2, SEQ ID NO:10, Oligo 3, SEQ ID NO:11.
**Figure 6****:** Shows: (1, upper) The location of the proximal guide RNA binding site (gRNA3, SEQ ID NO:12) and distal guide RNA binding site (gRNA1, SEQ ID NO:13) used for the CRISPR/Cas9 mediated knockout of the *Adam6a* gene in TRN0010 (See Fig. 4G for a schematic showing the partly human TRN0010 heavy chain locus containing *Adam6* genes) homozygous **zygotes. (The last 3 nucleotides at the 3' end of these sequences** correspond to the PAM required for Cas9 function. (2, lower) The structure of the locus after deletion of *Adam6a* and the location of the binding sites for the oligonucleotides used for confirmation of the *Adam6a* gene knockout in founder mice by genomic PCR. Oligo 1, SEQ ID NO:14, Oligo 13, SEQ ID NO:15, Oligo 3, SEQ ID NO:16.

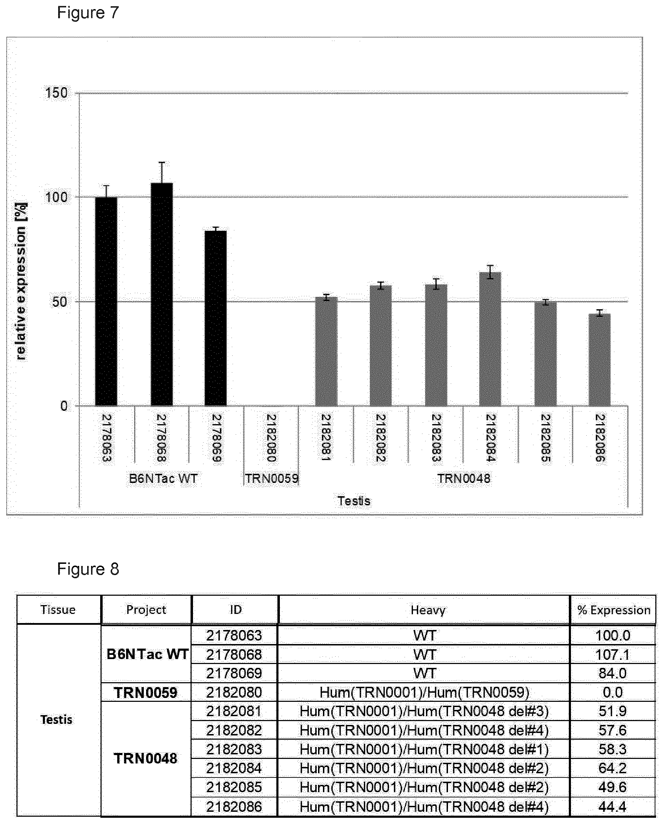

**Figure 7****:** Relative *Adam6a* expression in the testes of WT, null (TRN0059/TRN0001) and hemizygous (TRN0048/TRN0001) mice. (See Fig. 3G for a schematic showing the partly human TRN0001 heavy chain locus.) Graph bars illustrate the relative expression of *Adam6a* in the testes of wild type (*Adam6a*^{+/+}/*Adam6b*^{+/+}), *Adam6a*^{-/0}/*Adam6b*^{+/*0*} (TRN0059/TRN0001) and *Adam6a*^{+/0}/*Adam6b*^{-/0} (TRN0048/TRN0001) mice. Each bar represents the expression value of a mouse tissue sample compared to the first WT mouse tissue sample (#2178063, 100%).
**Figure 8****:** Percentage expression of *Adam6a* in the testes of wild type (WT), null (TRN0059/TRN0001) and hemizygous (TRN0048/TRN0001) mice relative to WT mouse 2178063. The percentage expression of *Adam6a* in the testes of WT (*Adam6a*^{+/+}/*Adam6b*^{+/+}), *Adam6a*^{*-*/*0*}/*Adam6b*^{+/*0*} (TRN0059/TRN0001) and *Adam6a*^{+/0}/*Adam6b*^{-/0} (TRN0048/TRN0001) mice is shown compared to the first WT mouse tissue sample (#2178063, 100%).

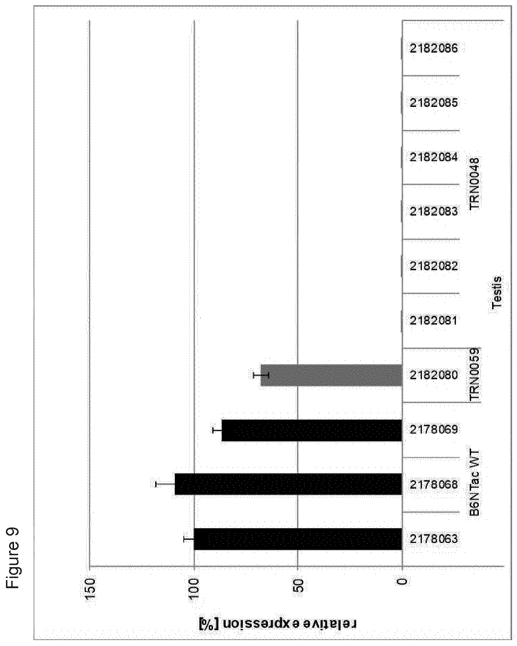

**Figure 9****:** Relative *Adam6b* expression in the testes of wild type (WT), hemizygous (TRN0059/TRN0001) and null (TRN0048/TRN0001) mice. (See Fig. 3G for a schematic showing the partly human TRN0001 heavy chain locus.) Graph bars Illustrate the relative expression of *Adam6b* in the testes of WT (*Adam6a*^{+/+}/*Adam6b*^{+/+}), *Adam6a*^{*-*/*0*}/*Adam6b*^{+/*0*} (TRN0059/TRN0001) and *Adam6a*^{+/0}/*Adam6b*^{-/0} (TRN0048/TRN0001) mice. Each bar represents the expression value of a mouse tissue sample compared to the first WT mouse tissue sample (#2178063, 100%).

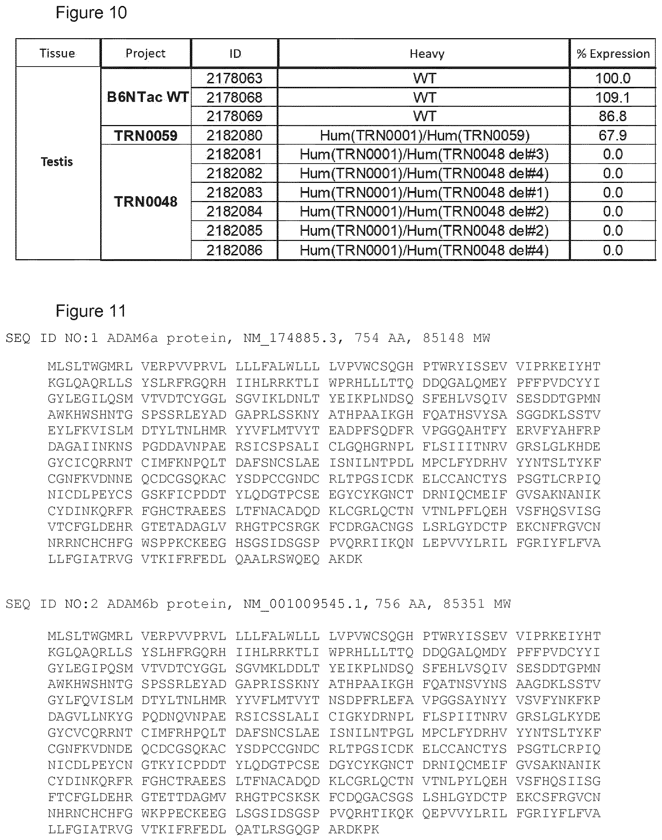

**Figure 10****:** Percentage expression of *Adam6b* in the testes of heterozygous (TRN0059/TRN0001) and null (TRN0048/TRN0001) mice relative to wild type (WT) mouse 2178063. The percentage expression of *Adam6b* in the testes of wild type (*Adam6a*^{+/+}/*Adam6b*^{+/+})*, Adam6a*^{*-*/*0*}/*Adam6b*^{+/*0*} (TRN0059/TRN0001) and *Adam6a*^{+/0}/*Adam6b*^{-/0} (TRN0048/TRN0001) mice is shown compared to the first WT mouse tissue sample (#2178063, 100%).

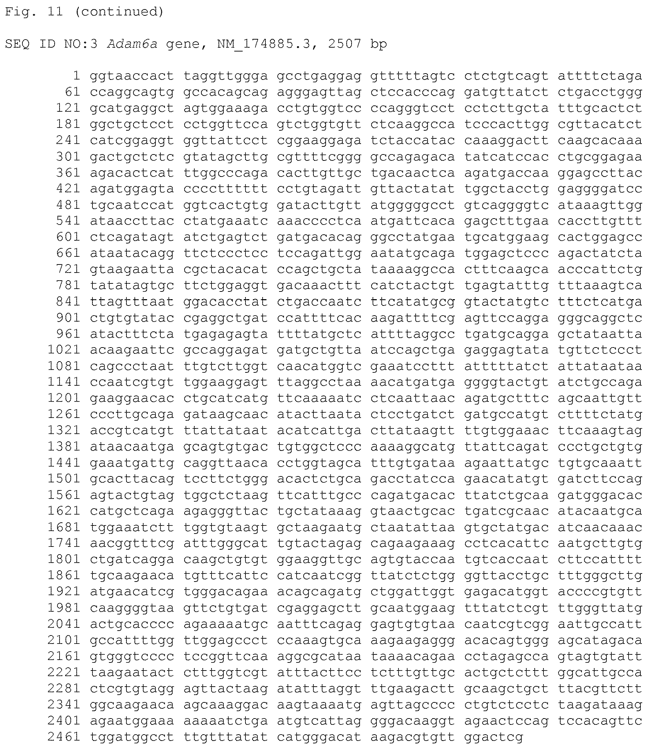

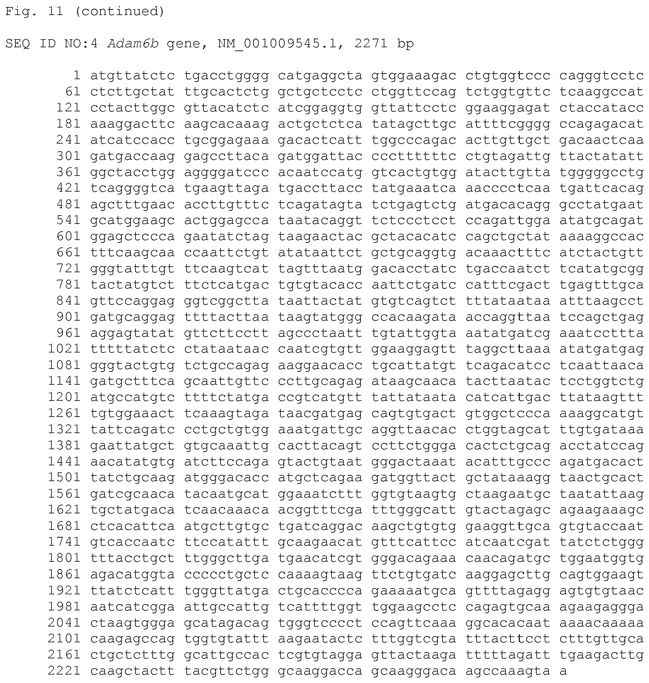

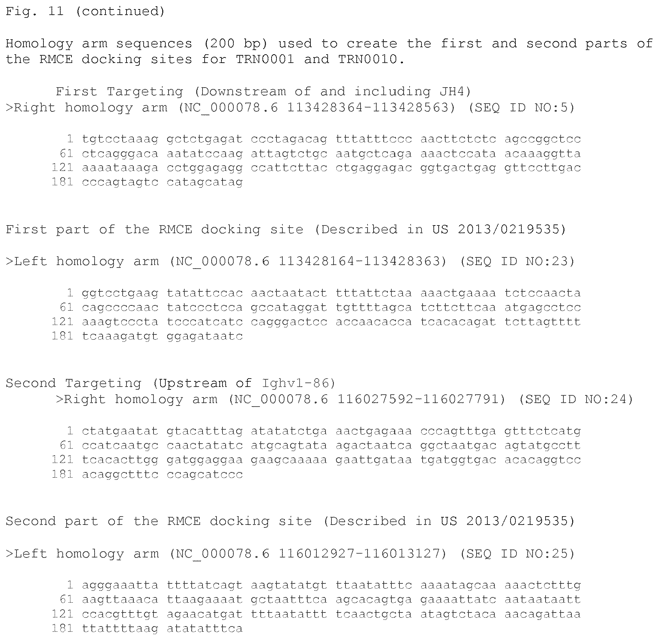

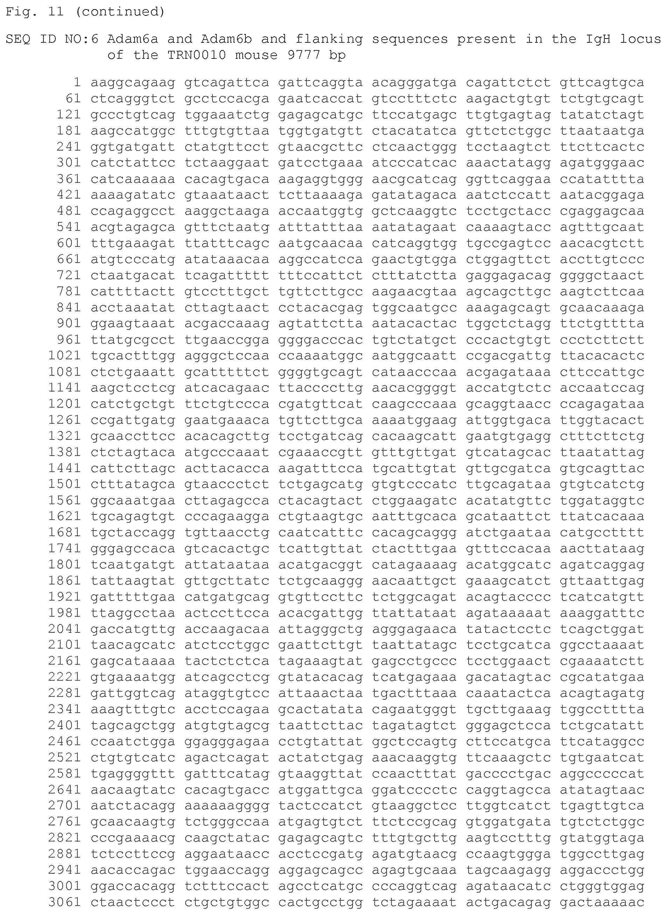

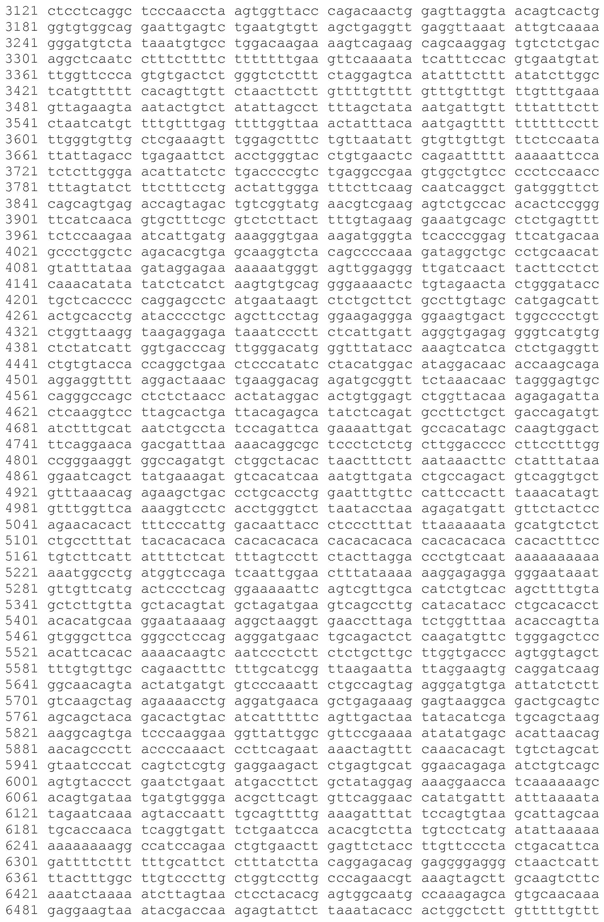

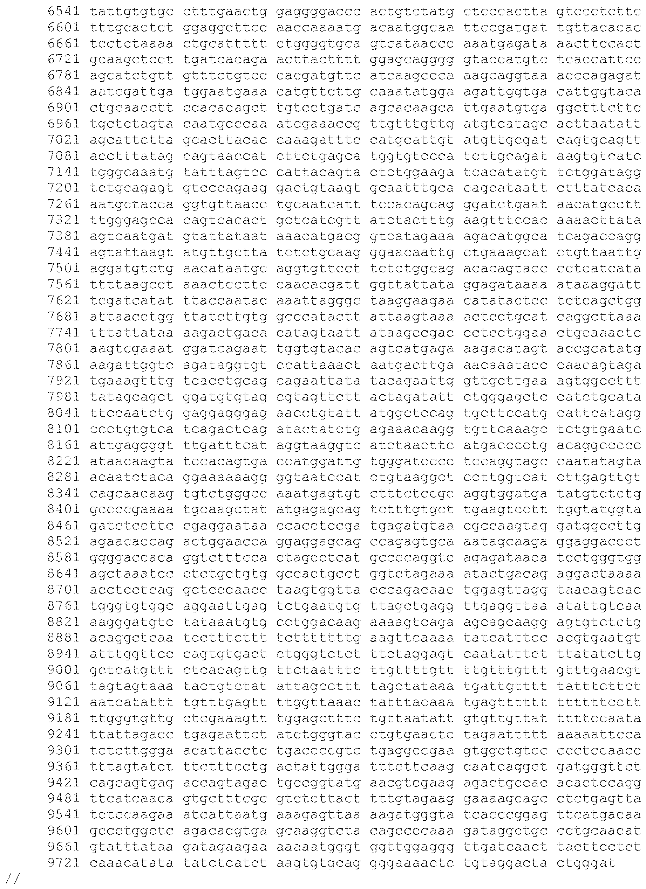

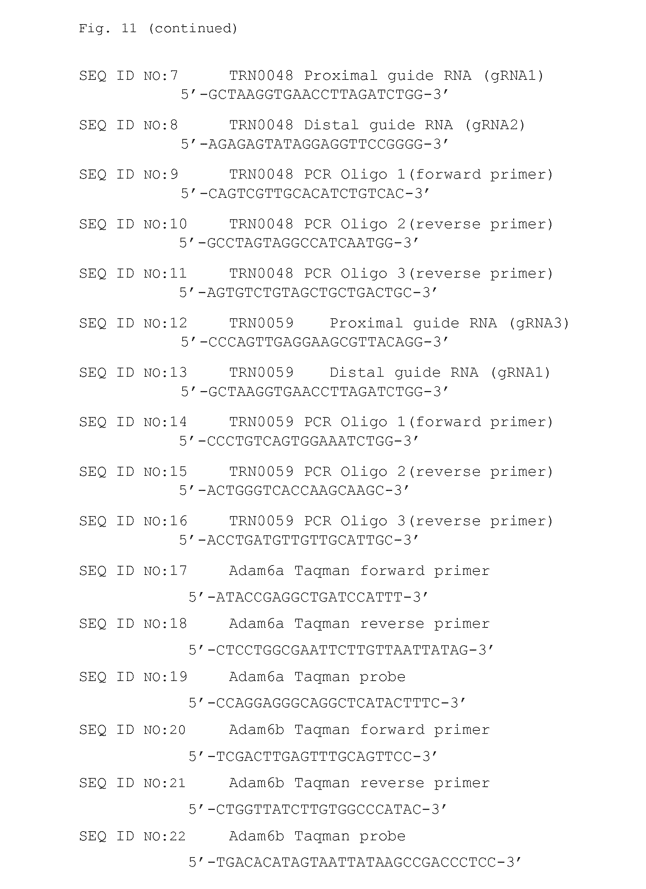

**Figure 11****:** Protein and amino acid sequences disclosed herein.

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (2nd Ed.), Vols. 1 -3, Cold Spring Harbor Laboratory Press (1989); Lewin, "Genes IV", Oxford University Press, New York, (1990), and Janeway et al., "Immunobiology" (5th Ed., or more recent editions), Garland Science, New York, 2001.

The terms "comprise", **"contain", "have" and "include" as used herein can** be used synonymously and shall be understood as an open definition, allowing further members or **parts or elements. "Consisting" is considered as a closest definition without further elements of the consisting definition feature. Thus "comprising" is broader** and contains the **"consisting" definition.**

**The term** "about" **as used herein refers to the same value or a value differing by +/-**5 % of the given value.

**The term** "ADAM6 protein" **shall particularly refer to** an ADAM6a or ADAM6b protein which is expressed by a wild-type mouse, or variants of the ADAM6a and ADAM6b protein, respectively, specifically those that have a certain sequence similarity to the respective native protein, and are expressed by an exogenous *Adam6* transgene in a transgenic mouse as described herein, and in particular those that are functional in a male mouse. An ADAM6 variant is understood to be functional in so far that its expression results in fertility in a male mouse. Functional variants may, *e.g*., be obtained by mutagenesis methods, in particular to delete, exchange, introduce inserts or deletions into a specific amino acid sequence or region or chemically derivatize an amino acid sequence, and those variants which are determined to be functional in a suitable assay be selected from engineering a transgenic mouse described herein. Any of the known mutagenesis methods may be employed, including point mutations at desired positions, *e.g*., obtained by randomization **techniques. The term "mutagenesis" refers** to any art-recognized technique for altering a polynucleotide or polypeptide sequence. Preferred types of mutagenesis include error prone PCR mutagenesis, saturation mutagenesis or site directed mutagenesis.

**The term "antibody" as used herein shall ref**er to polypeptides or proteins that consist of or comprise antibody domains in various combinations or constructions, e.g., constant and/or variable domains of the heavy and/or light chains of immunoglobulins, with or without linker sequences. Polypeptides are understood as antibody domains, if comprising a beta-barrel structure consisting of at least two beta-strands of an antibody domain structure connected by a loop sequence, and comprising an antigen-binding site, which is particularly formed by loop sequences such as complementarity-determining regions (CDRs). Antibody domains may be of native structure or modified by mutagenesis or derivatization, *e.g*., to modify the antigen binding properties or any other property, such as stability or functional properties, such as binding to Fc receptors.

**Herein, the term "antibody" and "immunoglobulin" are used interchangeably.**

**The term "antibody" as used herein shall particularly refer to antibody constructs** comprising a pair of a V_{H} (heavy chain variable domain) and a VL (light chain variable domain), thereby obtaining an antigen-binding site composed of the variable regions of a combination of three CDRs of a V_{H} and three CDRs of a VL. Alternatively, an antigen-binding site can be comprised in the variable region of a single V_{H}. One or more variable domains can be directly fused with constant antibody domains, and/or linked using one or more linking sequence(s) or hinge region(s). Antibodies may be full-length antibodies or antigen-binding fragments, e.g., heavy-chain only antibodies, composed of one or two single chains, wherein each single chain comprises or consists of a variable heavy chain region (or V_{H}) linked to one or more constant domains. Antibodies described herein may comprise or consist of antibody domains or the full-length structure of an IgG type (*e.g*., an IgG1, IgG2, IgG3, or IgG4 subtype), IgA1, IgA2, IgD, IgE, or IgM type, or their murine counterparts.

Specifically, an antibody is typically understood as a polypeptide or protein (or protein complex consisting of two or four antibody domain chains) that comprises an amino acid sequence encoded by immunoglobulin genes or fragments of immunoglobulin genes. The recognized immunoglobulin genes include the kappa, lambda, alpha, gamma, delta, epsilon, and mu constant region genes, as well as immunoglobulin variable region genes. Light chains (LC) are classified as either kappa or lambda. Heavy chains (HC) are classified as gamma, mu, alpha, delta, or epsilon, which in turn define the immunoglobulin classes, IgG, IgM, IgA, IgD and IgE, respectively.

In a typical IgG antibody structure, HC or LC each contain at least two domains connected to each other to produce a pair of antibody constant domains and binding site domains. In specific cases, a heavy chain may incorporate a LC constant domain, yet still be considered a HC, e.g., being devoid of a light chain variable domain or region.

The HC of an antibody may comprise a hinge region connecting one or more (e.g. two) antigen-binding parts of an antibody to an antibody Fc part. Exemplary antibody constructs may contain antibody constant domains, such as of an Fc connected through an antibody hinge region.

The hinge region may be a naturally-occurring heavy chain hinge region of an immunoglobulin, *e.g.*, of an IgG1 or an IgG3, or an artificial hinge region comprising or consisting of a number of consecutive amino acids which is of about the same length (+/-20%, or +/-10%) as a naturally-occurring one. Preferred hinge regions comprise one or more, *e.g.*, 2, 3, or 4 cysteine residues that may form disulfide bridges to another hinge region thereby obtaining a dimeric construct.

**The term** "antibody" **shall apply to** antibodies of animal origin, including human species, such as mammalian, including human, murine, rabbit, rat, or avian, such as chicken, which term shall particularly include recombinant antibodies that comprise one or more sequence(s) of human and/or non-human animal origin, *e.g.*, mouse sequences.

**Specifically, the term** "antibody" **applies to** antibodies produced by transgenic non-human animals, *e.g.*, from mice, which comprise human antigen-binding regions and non-human (*e.g*., murine) constant regions and/or framework sequences.

**The term** "antibody" **further applies to fully human** antibodies.

**The term** "fully **human" as used with respect to an immunoglobulin, is understood to** include antibodies having variable and having constant regions derived from human germline immunoglobulin sequences. A human antibody may include amino acid residues not encoded by human germline immunoglobulin sequences (*e.g*., mutations introduced by random or site-specific mutagenesis *in vitro* or by somatic mutation *in vivo*)*,* for example in the CDRs. Human antibodies include antibodies isolated from human immunoglobulin or antibody libraries or from animals transgenic for one or more human immunoglobulin genes.

A fully human or chimeric antibody or antibody fragment may be produced by transgenic mice, such as, *e.g*., the transgenic mouse described herein, comprising a chimeric immunoglobulin locus, e.g., an immunoglobulin heavy chain variable region locus comprising human immunoglobulin gene segment coding sequences and murine expression control sequences, wherein the murine expression control sequences are in operable linkage to control expression of the human heavy chain sequences, thereby producing an antibody comprising only human heavy chain sequences.

A human immunoglobulin is preferably selected or derived from the group consisting of IgA1, IgA2, IgD, IgE, IgG1, IgG2, IgG3, IgG4 and IgM.

A murine immunoglobulin is preferably selected or derived from the group consisting of IgA, IgD, IgE, IgG1, IgG2A, IgG2B, IgG2C, IgG3 and IgM.

**The term** "antibody" **further applies to chimeric antibodies, with mixed sequences that** originate from different species, such as sequences of murine and human origin.

**The term "chimeric" as used with respect to an immunoglobulin or an antibody refers** to those molecules wherein one portion of an antibody chain is homologous to corresponding sequences in immunoglobulins derived from a particular species or belonging to a particular class, while the remaining segment of the chain is homologous to corresponding sequences in another species or class. Typically, the variable region mimics the variable regions of immunoglobulins derived from one species of mammals, while the constant portions are homologous to sequences of immunoglobulins derived from another. For example, the variable region can be derived from presently known sources using human immunoglobulin gene segment coding sequences or readily available B-cells from human host organisms, and can be combined with one or more constant region(s) derived from, for example, non-human animal cell preparations. Specifically, chimeric antibodies or antibody fragment may be produced by transgenic mice, such as *e.g.* the transgenic mouse described herein, which comprise human variable region sequences (or the respective variable antibody domains) and mouse constant region sequences (or the respective constant region antibody domains).

The term "antibody" **further applies to a monoclonal antibody, specifically a** recombinant antibody, which term includes all types of antibodies and antibody structures that are prepared, expressed, created or isolated by recombinant means, such as antibodies originating from animals, *e.g.*, mammalians including human, that comprises genes or sequences from different origin, *e.g.*, chimeric, humanized antibodies, or hybridoma-derived antibodies. Further examples refer to antibodies isolated from a host cell transformed to express the antibody, or antibodies isolated from a recombinant, combinatorial library of antibodies or antibody domains, or antibodies prepared, expressed, created or isolated by any other means that involve splicing of antibody gene sequences to other DNA sequences.

**Herein the term "endogenous"** with reference to a nucleic acid element, e.g., a nucleic acid construct such as an immunoglobulin gene segment, or a non-coding or coding gene sequence, such as a gene, or locus, indicates that the nucleic acid element is comprised in a cell to be endogenous to said cell, wherein the cell is a native or wild-type cell, or wherein the nucleic acid element is present at a particular locus in the genome of a non-modified cell. An endogenous gene may be a wild type gene present at that locus in a wild type cell (as found in nature). An endogenous nucleic acid element may be a modified endogenous nucleic acid element if it is present at the same locus in the genome as a wild type nucleic acid element. An example of such a modified endogenous nucleic acid element is a gene into which a foreign nucleic acid is inserted. An endogenous gene may be present in the nuclear genome, mitochondrial genome etc.

**The term "exogenous" as used herein shall refer to those elements** that originate **from sources that are external to a certain cell, thus, not "endogenous" to such cell.** Specifically, the exogenous *Adam6* transgene, also called *Adam6* transgene, as described herein is exogenous to the mouse cell and can replace (non-functional or knocked out) endogenous *Adam6a* and *Adam6b* genes. Specifically, the exogenous *Adam6* transgene is comprised within a heterologous expression cassette comprising such *Adam6* transgene optionally besides other nucleic acid elements such as immunoglobulin gene segment coding sequences and mouse non-coding sequences. The exogenous *Adam6* transgene may be an ortholog or homolog of a closely related species with respect to the mouse, e.g., a rodent, such as a mouse of a different strain or species, or a rat of any species. The addition of the ortholog or homolog to the mouse rescues the loss of fertility due to loss of ADAM6 function.

**The term "expression cassette" as described herein** refers to nucleic acid molecules, elements or genetic constructs containing desired coding sequences in operable linkage, so that hosts comprising such expression cassette are capable of producing the encoded proteins. The expression system may be included in a vector to transform a host; however, the relevant coding sequence may also be directly integrated into the host chromosome.

Typically, the expression cassette comprises restriction sites that are designed to ensure insertion of the cassette in the proper reading frame. Foreign DNA can be inserted at one or more restriction sites of a vector DNA, and then be carried by the vector into a host cell along with the transmissible vector DNA.

Expression vectors typically comprise an expression cassette and additionally usually comprise an origin for autonomous replication in the host cells or a genome integration site, one or more selectable markers (e.g. an amino acid synthesis gene or a gene conferring resistance to antibiotics such as zeocin, kanamycin, G418 or hygromycin), a number of restriction enzyme cleavage sites, a suitable promoter sequence and a transcription terminator, which components are operably linked together. The term vector includes autonomously replicating nucleotide sequences as well as genome integrating **nucleotide sequences. A common type of vector is a "plasmid", which generally is a self-**contained molecule of double-stranded DNA that can be propagated in a bacterial host and readily accepts additional (foreign) DNA and which can readily be introduced into a suitable host cell. A plasmid vector often contains coding DNA and promoter DNA and has one or more restriction sites suitable for inserting foreign DNA. Specifically, the **term "vector" or "plasmid" refers to a vehicle by which a DNA or RNA sequence (e.g. a foreign gene) can** be introduced into a host cell, so as to transform the host and promote expression (e.g., transcription and translation) of the introduced sequence.

The expression cassette described herein specifically comprises an exogenous *Adam6* as described herein. Such expression cassette is specifically designed to incorporate the exogenous *Adam6* transgene into the mouse genome, to enable its expression *in vivo.*

As described herein, expression of the endogenous Adam6a and Adam6b genes is **reduced by one or more "genetic modifications". Specifically, reduction of expression of** endogenous *Adam6a* and *Adam6b* may also refer to complete reduction or elimination of expression of the endogenous *Adam6* genes.

Specifically, said one or more genetic modifications comprises a disruption, substitution, deletion or knockout of (i) one or more endogenous polynucleotides, or a part thereof; or (ii) an expression control sequence, preferably the expression control sequence is selected from the group consisting of a promoter, transcriptional start and stop sequences, enhancer and activator sequences, a ribosome binding site, or translational start and stop sequences.

A genetic modification may comprise partial deletion or full deletion, also referred to **as "knock-out", of the** *Adam6a* and *Adam6b* genes or inactivating mutations, also referred **to as "loss-of-function" mutations including e.g. point mutations or frameshift mutations.** An inactivating mutation may comprise a sequence modification to alter one or more contiguous amino acids, *e.g.* 1, 2, or 3 amino acids, which are substituted, inserted or deleted at one position in an amino acid sequence expressed by the respective gene. An inactivating mutation may also refer to areas of sequences, in particular changing contiguous or non-contiguous amino acid sequences.

The genetic modification may comprise a replacement, wherein a nucleic acid element is placed into the mouse genome by a heterologous nucleic acid element at the locus of the genomic sequence. The DNA sequence so placed may include one or more regulatory sequences that are part of the source DNA used to obtain the sequence so placed (e.g., promoters, enhancers, 5'- or 3'-untranslated regions, appropriate recombination signal sequences, etc.). For example, in various embodiments, the replacement is a substitution of an endogenous sequence for a heterologous sequence that results in the production of a gene product from the DNA sequence so placed (comprising the heterologous sequence), but not expression of the endogenous sequence. In a specific embodiment, an immunoglobulin gene segment is replaced with a corresponding human immunoglobulin gene segment. In another specific embodiment, endogenous immunoglobulin gene segment coding sequences are replaced with corresponding human immunoglobulin gene segment coding sequences, such as an ortholog of, a homolog of endogenous sequences, or those that are at least 90% or 95%, or 100% identical to the endogenous sequences that are replaced. In yet another specific embodiment, endogenous immunoglobulin gene segment coding sequences are replaced with corresponding human immunoglobulin coding sequences embedded in mouse non-coding and regulatory sequences.

**Herein, the term "heterologous" with reference to a** nucleic acid element, *e.g.*, a nucleic acid construct such as an immunoglobulin gene segment, or a non-coding or coding gene sequence, such as a gene, or locus, indicates that the gene, gene segment, and locus, respectively, is not native to a cell (*i.e.*, not natively occurring in a wild-type cell of the same species at the same location within the cellular genome), or foreign to a cell to produce a recombinant cell, *i.e.*, the nucleic acid element is present in the genome of a modified (recombinant) cell which is not a wild-type cell. A heterologous gene segment may be a wild type gene segment present at a locus which is different from the respective locus in a wild type cell (thus not found in nature at the same locus). A heterologous gene segment may comprise a (modified or unmodified) endogenous coding sequence or gene, if it is present at a different locus in the genome other than found in a wild type gene or organism. An example of such heterologous nucleic acid element is a modified endogenous one, such as the immunoglobulin heavy chain variable region (IHVR) locus described herein, comprising the *Adam6* transgene described herein, human immunoglobulin gene segment (hIGS) coding sequences and murine expression control sequences.

**The term "host" as used herein** with regard to a cell, cell line, or higher organism such as non-human animals, and any progeny of any of the foregoing, shall refer to a primary subject transformed to produce a particular recombinant protein, such as an antibody. It should be understood that not all progeny are exactly identical to the parental cell (due to deliberate or inadvertent mutations or differences in environment), however, such altered progeny are included in these terms, so long as the progeny retain the same **functionality as that of the originally transformed cell. The term "host cell line" refers to a** cell line of host cells as used for expressing a recombinant gene to produce recombinant **polypeptides such as recombinant antibodies. The term "cell line" as used herein refers to** an established clone of a particular cell type that has acquired the ability to proliferate over a prolonged period of time. Such host cell or host cell line may be maintained in cell culture and/or cultivated to produce a recombinant polypeptide.

The antibodies produced by a transgenic mouse described herein are specifically used as a template to provide antibody sequences e.g. of the antigen-binding site and in particular the CDR sequences. Such sequences can be used to provide coding nucleic acid molecules to engineer recombinant host cells for the producing recombinant antibodies comprising said antigen-binding site and CDRs, respectively.

**The term "isolated" or "isolation" as used herein with respect to** a compound such as a nucleic acid, a nucleic acid element, or an antibody shall refer to such compound that has been sufficiently separated from the environment with which it would naturally be **associated, so as to exist in "substantially pure" form. "Isolated" does not necessarily mean** the exclusion of artificial or synthetic mixtures with other compounds or materials, or the presence of impurities that do not interfere with the fundamental activity, and that may be present, for example, due to incomplete purification. In particular, isolated nucleic acid molecules as described herein are also meant to include those chemically synthesized.

With reference to nucleic acids or a nucleic acid elements as described herein, the **term "isolated",** when applied to DNA, refers to a DNA molecule that is separated from sequences with which it is immediately contiguous in the naturally occurring genome of the **organism in which it originated. For example, an "isolated nucleic acid" may comprise a** DNA molecule inserted into an expression cassette or a vector, such as a plasmid or virus vector, or integrated into the genomic DNA of a prokaryotic or eukaryotic cell or host **organism. When applied to RNA, the term "isolated" refers primarily to an RNA molecule** encoded by an isolated DNA molecule as defined above. Alternatively, the term may refer to an RNA molecule that has been sufficiently separated from other nucleic acids with which it would be associated in its natural state (*i.e.*, **in cells or tissues). An "isolated nucleic acid"** (either DNA or RNA) may further represent a molecule produced directly by biological or synthetic means and separated from other components present during its production.

With reference to polypeptides or proteins, such as isolated antibodies, the term **"isolated" shall specifically refer to compounds that are free or substantially free of material** with which they are naturally associated such as other compounds with which they are found in their natural environment, or the environment in which they are prepared (eg., cell culture) when such preparation is by recombinant DNA technology practiced *in vitro* or *in vivo.* Isolated compounds can be formulated with diluents or adjuvants and still for practical purposes be isolated - for example, the polypeptides or polynucleotides can be mixed with pharmaceutically acceptable carriers or excipients when used in diagnosis or therapy.

Antibodies generated using the transgenic mouse described herein are particularly provided in the isolated form, which are substantially free of other antibodies directed against different target antigens and/or comprising a different structural arrangement of antibody domains. Still, an isolated antibody may be comprised in a combination preparation, containing a combination of the isolated antibody, *e.g.*, with at least one other antibody, such as monoclonal antibodies or antibody fragments having different specificities.

**The term "locus" as used herein** refers to a DNA coding sequence or segment of DNA that codes for an expression product, which is a genomic sequence, such as part of (or isolated from) a genome of a host organism, or which is part of a vector, and is placed **within the host organism's genome at a certain chromosomal site, or can be** integrated, *e.g.*, at a predetermined target site, such as by defined restriction sites, or regions of homology by homologous recombination.

Restriction sites can be designed to ensure insertion of an expression cassette in the proper reading frame. Typically, foreign (herein also referred to as exogenous) DNA is inserted at one or more restriction sites of a vector DNA, and then is carried by the vector into a host cell along with the transmissible vector DNA.

Typically, a locus encompasses at least one gene or one or more gene segment(s). **The term "locus"** does not imply that a gene is actively transcribed or intact. Genes may be encompassed that have been inactivated.

A specific example of a locus described herein is an immunoglobulin heavy chain variable region (IHVR) locus in a mouse, comprising an exogenous *Adam6* transgene as described herein. Specifically, the IHVR locus comprises a first modification which results in the reduction or elimination of expression of endogenous ADAM6a and ADAM6b protein in a male mouse and a second modification comprising inserting an exogenous *Adam6* transgene as described herein. The first and second modifications of the locus can be made simultaneously or sequentially.

A further specific example of a locus described herein is an immunoglobulin heavy chain variable region (IHVR) locus in a mouse, comprising an exogenous *Adam6* transgene as described herein, human immunoglobulin gene segment (hIGS) coding sequences and murine expression control sequences. Specifically, the hIGS coding sequences are of immunoglobulin heavy chain variable region (IGHV), diversity (IGHD) and joining (IGHJ) gene segments.

A locus may be engineered to express exons encoding an antibody or one or more antibody domains, such as described herein.

A recombinant locus can be created using various conventional techniques for site-specific editing and/or recombination. Preferably, a modified locus is generated by inserting **a piece of DNA (referred to here as the "donor DNA") containing gene segments encoding,** *e*.*g*., human immunoglobulin gene segments or coding sequences of human immunoglobulin gene segments, into a modified version of a murine immunoglobulin locus such as a heavy chain locus of a host organism (herein **referred to as the "acceptor allele").** The acceptor allele may contain recognition sites for a site-specific DNA recombinase, such as the Cre recombinase (a IoxP site and a mutated version of the IoxP site). The donor DNA may be flanked by the same Cre recombinase recognition sites (**at both, the 5'**-end and the **3'**-end, *e.g.*, on one side there is a IoxP site and on the other there will be a mutated version of the IoxP site). The Cre recombinase may be used to catalyze the insertion of the donor DNA into the acceptor allele in a process called Recombinase Mediated Cassette Exchange (RMCE).

Introduction of the site-specific recombination sites may also be achieved by conventional homologous recombination techniques. Such techniques are described in references such as *e.g*., Sambrook and Russell (2001) Molecular cloning: a laboratory manual, 3d ed. (Cold Spring Harbor, N.Y.: Cold Spring Harbor Laboratory Press); Nagy, (2003) Manipulating the mouse embryo: a laboratory manual, 3d ed. (Cold Spring Harbor, N.Y.: Cold Spring Harbor Laboratory Press); Renault and Duchateau, Eds. (2013) Site-directed insertion of transgenes. (Topics in Current Genetics 23, Springer); and Tsubouchi, H. Ed. (2011) DNA recombination, Methods and Protocols. (Humana Press).

In a specific embodiment, nucleic acid sequences and gene segments can be introduced into an immunoglobulin locus by homologous recombination. In such an embodiment, targeting sequences or vectors are employed that are comprised of genomic targeting homology arms flanking a nucleic acid sequence comprising antibody encoding gene segments (*i.e*., **a nucleotide sequence at both, the 5'-end and the 3'**-end, which is homologous to and capable of hybridizing with a target sequence). These genomic homology arms facilitate insertion of the antibody encoding DNA into an immunoglobulin locus, such as DNA that encodes the immunoglobulin heavy chain. The targeting sequence refers to a sequence that is homologous to DNA sequences in the genome of a cell that flank or occur adjacent to the region of an immunoglobulin genetic locus that is to be modified. The flanking or adjacent sequence may be within the locus itself or upstream or downstream of coding sequences in the genome of the host cell. Targeting sequences are inserted into recombinant DNA vectors for use in cell transfections such that sequences to be inserted into the cell genome, such as the sequence of a recombination site, are flanked by the targeting sequences of the vector.

In many instances in which homologous recombination is employed to accomplish a genetic change in a genome (such as an insertion or a deletion), a further modification would involve the use of engineered site-specific endonucleases to increase the likelihood that a desired outcome can be accomplished. Such endonucleases are of value because they can be engineered to be highly specific for unique sequences in a target genome, and because they cause double-stranded DNA breaks at the sites they recognize. Double-stranded breaks promote homologous recombination with targeting vectors that carry targeting homology with DNA in the immediate vicinity of the breaks. Thus, the combination of a targeting vector and a site-specific endonuclease that cleaves DNA within or close to the region targeted by a vector typically results in much higher homologous recombination efficiency than use of a targeting vector alone. Furthermore, it is possible to facilitate the creation of a genomic deletion through use of one or more site-specific endonucleases and a targeting vector comprised of two targeting homology arms in which one arm targets one side of the region to be deleted and the other arm targets the other side.

Site-specific recombination differs from general homologous recombination in that short specific DNA sequences, which are required for the recombinase recognition, are the only sites at which recombination occurs. Site-specific recombination requires specialized recombinases to recognize the sites and catalyze the recombination at these sites. A number of bacteriophage and yeast-derived site-specific recombination systems, each comprising a recombinase and specific cognate target sites, have been shown to work in eukaryotic cells for the purpose of DNA integration and are therefore applicable for use as described herein. These include the bacteriophage P1 Cre/lox, yeast FLP-FRT system, and the Dre system of the tyrosine family of site-specific recombinases. Such systems and methods of use are well-described in the prior art. The recombinase-mediated cassette exchange (RMCE) procedure is facilitated by usage of the combination of wild-type and mutant IoxP (or FRT, etc.) sites together with the appropriate recombinase (*e.g*., Cre or Flp), and negative and/or positive selection. RMCE will occur when the sites employed are identical to one another and/or in the absence of selection, but the efficiency of the process is reduced because excision rather than insertion reactions are favored and, without incorporating positive and/or negative selection, there will be no enrichment for appropriately mutated cells.

Other systems of the tyrosine family of site-specific recombinases, such as bacteriophage lambda Int integrase and HK2022 integrase, and in addition systems belonging to the separate serine family of recombinases such as bacteriophage phiC31, R4Tp901 integrases are known to work in mammalian cells using their respective recombination sites, and are also applicable for use as described herein.

The methods described herein specifically utilize site-specific recombination sites that utilize the same recombinase, but which do not facilitate recombination between the sites. For example, a IoxP site and a mutated IoxP site can be integrated into the genome of a host, but introduction of Cre into the host will not cause the two sites to undergo recombination; rather, the IoxP site will recombine with another IoxP site, and the mutated site will only recombine with another likewise mutated IoxP site.

Two classes of variant recombinase sites are available to facilitate recombinase-mediated cassette exchange. One harbors mutations within the 8 bp spacer region of the site, while the other has mutations in the 13-bp inverted repeats.

Spacer mutants such as Iox511 (Hoess, et al., Nucleic Acids Res., 14:2287-00 (1986)), Iox5171 and lox2272 (Lee and Saito, Gene, 216:55-65 (1998)), m2, m3, m7, and m11 (Langer, et al., Nucleic Acids Res., 30:3067-77 (2002)) recombine readily with themselves but have a markedly reduced rate of recombination with the wild-type site. Examples of the use of mutant sites of this sort for DNA insertion by recombinase-mediated cassette exchange can be found in Baer and Bode, Curr. Opin. Biotechnol., 12:473-80 (2001).

Inverted repeat mutants represent a second class of variant recombinase sites. For example, IoxP sites can contain altered bases in the left inverted repeat (LE mutant) or **the right inverted repeat (RE mutant). An LE mutant, Iox71, has 5 bp on the 5' end of the** left inverted repeat that are changed from the wild type ATAAC sequence to TACCG (Araki, Nucleic Acids Res., 25:868-72 (1997); Missirlis, et al., BMC Genomics, 7:73 (2006)). **Similarly, the RE mutant, Iox66, has the five 3'**-most bases changed from GTTAT to CGGTA. Inverted repeat mutants can be used for integrating plasmid inserts into **chromosomal DNA. For example, the LE mutant can be used as the "target" chromosomal IoxP site into which the "donor" RE mutant recombines. After recombination a donor piece** of DNA that contained an RE site will be found inserted in the genome flanked on side by a double mutant site (containing both the LE and RE inverted repeat mutations) and on the other by a wild-type site (Lee and Sadowski, Prog. Nucleic Acid Res. Mol. Biol., 80: 1-42 (2005)). The double mutant is sufficiently different from the wild-type site that it is unrecognized by Cre recombinase and the inserted segment therefore cannot be excised by Cre-mediated recombination between the two sites.

In certain aspects, site-specific recombination sites can be introduced into introns or intergenic regions, as opposed to coding nucleic acid regions or regulatory sequences. This may avoid inadvertently disrupting any regulatory sequences or coding regions necessary for proper gene expression upon insertion of site-specific recombination sites into the genome of the animal cell.

Specific recombination into the genome can be facilitated using vectors designed for positive or negative selection as known in the art. In order to facilitate identification of cells that have undergone the replacement reaction, an appropriate genetic marker system may be employed and cells selected by, for example, use of a selection medium. However, in order to ensure that the genome sequence is substantially free of extraneous nucleic acid sequences at or adjacent to the two end points of the replacement interval, desirably the marker system/gene can be removed following selection of the cells containing the replaced nucleic acid.

The recombinase may be provided as a purified protein, or may be expressed from a construct transiently expressed within the cell in order to provide the recombinase activity. Alternatively, the cell may be used to generate a transgenic animal, which may be crossed with an animal that expresses said recombinase, in order to produce progeny that lack the marker gene and associated recombination sites.

In a specific embodiment, gene segments or respective immunoglobulin coding sequences can be introduced into an immunoglobulin locus by a CRISPR/Cas9 technology using a non-homologous end joining approach, *e.g.,* see He et al. (supra) or by using the CRISPR-associated transpose-mediated DNA insertion system (Strecker, et al., supra; Klompe, et al, supra).

**The term "sequence identity" as used herein is understood as the** relatedness between two amino acid sequences or between two nucleotide sequences and described by the degree of sequence identity. The sequence identity of a variant, homologue or orthologue as compared to a parent nucleotide or amino acid sequence indicates the degree of identity of two or more sequences. Two or more amino acid sequences may have the same or conserved amino acid residues at a corresponding position, to a certain degree, up to 100%. Two or more nucleotide sequences may have the same or conserved base pairs at a corresponding position, to a certain degree, up to 100%.

Sequence similarity searching is an effective and reliable strategy for identifying homologs with excess (*e.g.,* at least 50%) sequence identity. Sequence similarity search tools frequently used are *e.g.,* BLAST, FASTA, and HMMER. Sequence similarity searches can identify such homologous proteins or polynucleotides by detecting excess similarity, and statistically significant similarity that reflects common ancestry. Homologues may encompass orthologues, which are herein understood as the same protein in different organisms, e.g., variants of such protein in different different organisms or species.

**"Percent (%) identity" with respect to an amino acid sequence** of homologs and orthologues described herein is defined as the percentage of amino acid residues in a candidate sequence that are identical with the amino acid residues in the specific polypeptide sequence, after aligning the sequence and introducing gaps, if necessary, to achieve the maximum percent sequence identity, and not considering any conservative substitutions as part of the sequence identity. Those skilled in the art can determine appropriate parameters for measuring alignment, including any algorithms needed to achieve maximal alignment over the full length of the sequences being compared.

For purposes described herein, the sequence identity between two amino acid sequences is determined using the NCBI BLAST program version 2.9.1 (Apr-19-2019) with BLASTp set at the default parameters.

"Percent (%) identity" with respect to a nucleotide sequence *e.g.*, of a nucleic acid molecule or a part thereof, in particular a coding DNA sequence, is defined as the percentage of nucleotides in a candidate DNA sequence that is identical with the nucleotides in the DNA sequence, after aligning the sequence and introducing gaps, if necessary, to achieve the maximum percent sequence identity, and not considering any conservative substitutions as part of the sequence identity. Alignment for purposes of determining percent nucleotide sequence identity can be achieved in various ways that are within the skill in the art, for instance, using publicly available computer software. Those skilled in the art can determine appropriate parameters for measuring alignment, including any algorithms needed to achieve maximal alignment over the full length of the sequences being compared.

Optimal alignment may be determined with the use of any suitable algorithm tor aligning sequences, non-limiting examples of which include the Smith-Waterman algorithm, the Needleman-Wunsch algorithm, algorithms based on the Burrows-Wheeler Transform (e.g., the Burrows Wheeler Aligner), ClustalW, ClustalX, BLAT, Novoalign (Novocraft Technologies; available at novocraft.com), ELAND (Illumina, San Diego, CA), and Maq (available at maq.sourceforge.net).

The term "recombinant" refers to a polynucleotide (nucleic acid element) or polypeptide that does not naturally occur in a host cell or animal. A recombinant molecule may contain two or more naturally-occurring sequences that are linked together in a way that does not occur naturally. A recombinant cell contains a recombinant polynucleotide or polypeptide. If a cell receives a recombinant nucleic acid, the nucleic acid is "exogenous" to the cell and to the animal comprising said cells.

**The term "recombinant"** particularly means **"being prepared by or the result of genetic engineering". Alternatively, the term "engineered" is used.**

**The term "transgene" is used herein to describe genetic** material that has been or is about to be artificially inserted into the genome of a cell, and particularly a cell of a host **animal. The term "transgene" as used herein refers to a** nucleic acid molecule or element, *e.g.*, a nucleic acid comprised in an expression construct and/or a targeting vector to be **expressed in a host organism upon its introduction and incorporation into the host's** genome. **Thereby, the host organism is engineered as a "transgenic" host.**

**As used herein, the term** *"Adam6* transgene" **refers to the coding sequence of a** nucleotide sequence encoding a protein comprising a certain sequence identity to SEQ ID NO:1 and/or SEQ ID NO:2. The coding sequence may be artificial or of animal origin, specifically a mouse gene, or coding cDNA.

The term "transgenic animal" is herein understood as a non-human animal, usually a mammal, *e.g.*, a rodent, particularly a mouse or rat, although other mammals are envisioned, having an exogenous nucleic acid sequence present as a chromosomal or extrachromosomal element in a portion of its cells or stably integrated into its germ line DNA (*i.e.,* in the genomic sequence of most or all of its cells).

In certain aspects, the transgenic animals described herein comprise one or more nucleic acid molecule(s) or element(s) encoding human immunoglobulin regions. Numerous methods have been developed for replacing endogenous mouse immunoglobulin regions with human immunoglobulin sequences to create partially- or fully-human antibodies for drug discovery purposes. Examples of such mice include those described in, for example, U.S. Pat Nos. 7,145,056; 7,064,244; 7,041,871; 6,673,986; 6,596,541; 6,570,061; 6,162,963; 6,130,364; 6,091,001; 6,023,010; 5,593,598; 5,877,397; 5,874,299; 5,814,318; 5,789,650; 5,661,016; 5,612,205; and 5,591,669.

According to a specific aspect, the transgenic cells and animals described herein have a genome in which part or all of the endogenous immunoglobulin region is removed.

In another favored aspect, the **animal's** genome is modified so that its B cells are capable of expressing more than one functional V_{H} domain per cell, *e.g.*, producing bispecific antibodies comprising two different V_{H} domains and two different antigen-binding sites, respectively, such as described in WO2017035252A1.

In the particularly favored aspects, the transgenic animals described herein comprise one or more nucleic acid molecule(s) or element(s) that are chimeric immunoglobulin gene segments encoding IGHV, IGHK and IGHL as described in US20130219535. Such transgenic animals have a genome comprising an introduced nucleic acid element which is a partly human immunoglobulin region, wherein the introduced nucleic acid element comprises human variable region coding sequences and non-coding sequences based on the endogenous genome of the non-human vertebrate.

According to a specific embodiment a pharmaceutical composition is provided, comprising an antibody or antibody fragment derived from the transgenic mouse described herein, such as for example a full-length antibody, a heavy chain only antibody, **a** F**(ab**')2 fragment, a Fab fragment, or a single chain variable fragment (*e.g.* a scFV). A pharmaceutical composition can be provided which comprises an antibody and a pharmaceutically acceptable carrier or excipient in a formulation suitable for a specific administration route. Pharmaceutical compositions can be administered, *e.g.*, as a bolus injection, infusion or by continuous infusion. Exemplary formulations as used for parenteral administration include those suitable for subcutaneous, intramuscular or intravenous injection as, for example, a solution, emulsion or suspension. Pharmaceutical carriers suitable for facilitating such means of administration are well-known in the art.

Pharmaceutically acceptable carriers generally include any and all suitable solvents, dispersion media, coatings, isotonic and absorption delaying agents, and the like that are physiologically compatible with an antibody. Further examples of pharmaceutically acceptable carriers include sterile water, saline, phosphate buffered saline, dextrose, glycerol, ethanol, and the like, as well as combinations of any thereof.

Additional pharmaceutically acceptable carriers are known in the art and described in, e.g., Remington: The Science and Practice of Pharmacy, 22nd revised edition (Allen Jr, LV, ed., Pharmaceutical Press, 2012). Liquid formulations can be solutions, emulsions or suspensions and can include excipients such as suspending agents, solubilizers, surfactants, preservatives, and chelating agents.

The foregoing description will be more fully understood with reference to the following examples. Such examples are, however, merely representative of methods of practicing one or more embodiments of the present invention and should not be read as limiting the scope of invention.

### EXAMPLES

In mice, there are two ADAM6 proteins, ADAM6a and ADAM6b (SEQ ID NO:1 and 2), encoded by two single-exon intronless genes that are both expressed, as detected by gene-specific RT-PCR [Choi, I, et al., Genomics (2004) 83:636-646]. The *Adam6a* and *Adam6b* genes (SEQ ID NO:3 and 4) are located on chromosome 12 within the immunoglobulin heavy chain locus (*Igh*) between the *Ighv5-1p* and *Ighd1-1* gene segments but in opposite transcriptional orientation to the V_{H} gene segments [Featherstone, K, JBC (2010) 285:9327-9338] This is shown schematically for the TRN0010 *Igh* locus in Figure 2. (See below and Fig. 4 for a description of the TRN0010 mouse.) The *Adam6a* and *Adam6b* genes are 94% identical and the proteins are 90% identical.

Demonstrated herein is the discovery that only one exogenous *Adam6* transgene, i.e., only one of *Adam6a* or *Adam6b* or a functionally active variant thereof, is required for full male fertility.

Two existing Trianni mouse strains, TRN0001 and TRN0010, were used in the experiments described in the Examples. These mice were generated using conventional technologies well known in the art. For TRN0001 mice, the endogenous mouse IHVR locus was deleted and replaced by a synthetic DNA construct containing human IGHV, IGHD and IGHJ gene coding segments embedded in mouse regulatory and flanking sequences. No functional mouse *Adam6* genes were included in the construct. This mouse is described in detail in US 2013/0219535 and was generated using Recombinase Mediated Cassette Exchange (RMCE) as outlined in Fig. 3. (A) Schematic of the endogenous mouse IHVR allele in ES cells showing Ighv (V_{H} gene segments), *Adam6a* and *Adam6b* genes, Ighd (D_{H} gene segments), Ighj (J_{H} gene segments), and the Ighm (IgM constant region gene). (B) The same allele after insertion of the first part of the recombination docking site construct (1) downstream of the most proximal (relative to Ighm) Ighj gene segment by homologous recombination. (C) The same allele as in (B) after insertion of the second part of the recombination site docking construct (2) upstream of the most distal Ighv gene segment by homologous recombination. (SEQ ID NO:5 shows the sequence flanking the recombination sites (200 bp) for TRN0001.) (D) The configuration of the allele after *in vitro* deletion of the endogenous IHVR locus by Cre recombinase (3, FRT site; 4, Iox5171 site; 5, IoxP site) These sites are contained in the docking constructs (1, 2 in Fig. 3), which are described in detail in US 2013/0219535. The Cre-mediated deletion creates the recombination docking site (6), which is also described in detail in US 2013/0219535, to be used for RMCE. Recent publications describing RMCE and docking sites include Turan, S. Gene (2013) 515:1-27 and Pieters, T. J. Vis. Exp. (2017) 122:e55575, doi:10.3791/55575. (E) Schematic of the synthetic RMCE vector containing human IGHV, IGHD and IGHJ coding sequences embedded in mouse flanking and regulatory sequences. A neomycin resistance gene (Neo^{R}) **is contained at the 5' end of the vector for positive selection purposes. (F) The** targeted humanized IHVR allele in ES cells after successful RMCE. The modified ESC clones were validated for the correct insertion of the human synthetic locus by Southern Blot and/or Pulse Field Gel Electrophoresis (G) The final allele configuration after removal of the Neo^{R} gene by *in vivo* Flp recombination. Note the absence of any Adam6 genes. Validated ESC clones were injected into blastocysts and the resulting chimeras were bred in order to obtain heterozygous and ultimately homozygous mice Details about these methods can be found in US 2013/0219535.

The TRN0010 mouse was generated in the same way as the TRN0001 mouse except that the synthetic RMCE vector contained mouse *Adam6a* and *Adam6b* genes located between the most proximal IGHV and the most distal IGHD gene segments (SEQ ID NO:6). The method is outlined in Fig. 4. Note the presence of transgenic mouse *Adam6* genes, both *Adam6a* and *Adam6b,* in the final TRN0010 IgH allele configuration after removal of the Neo^{R} gene by *in vivo* Flp recombination (Fig. 4G).

### Example 1: Disruption of the Adam6b gene in TRN0010 zygotes using CRISPR/Cas9.

*Overview of the Adam6b targeting strategy.* According to NCBI and Ensembl databases, only one transcript exists for the *Adam6b* gene. The targeting strategy was based on NCBI transcript NM_001009545.1, which corresponds to Ensembl transcript ENSMUST00000063317.3. Figure 5 illustrates the location of the *Adam6a* and *Adam6b* genes within the TRN0010 *Igh* locus (top) and the expected configuration of the locus after deletion of the *Adam6b* gene (bottom). TRN0010 mice are homozygous for humanized IGH, IGK and IGL alleles and contain synthetic mouse *Adam6a* and *Adam6b* genes in their normal location on chromosome 12. Also indicated are the locations of the guide RNA binding sites, proximal (gRNA1, SEQ ID NO:7) and distal (gRNA2, SEQ ID NO:8), that were used in the CRISPR/Cas9-mediated deletion of *Adam6b.* These two guide RNAs were selected based on their position relative to *Adam6b* and the low number of potential off-targets. There were no sequences present in the TRN0010 mouse genome that were 100% identical to gRNA1 or gRNA2. The off-target analysis was based on the NCBI BLASTn analysis of the GRCm38/mm10 mouse genome assembly [UCSC Genome Browser assembly ID: mm10; Sequencing/Assembly provider ID: Genome Reference Consortium Mouse Build 38 (GCA_000001635.2); Assembly date: Dec. 2011; Accession ID: GCA_000001305.2] and the TRN0010 *Igh* allele sequence. This targeting strategy allowed for the generation of constitutive knockout of the Adam6b gene within the TRN0010 *Igh* locus via CRISPR/Cas9-mediated gene editing. Heterozygous (*Adam6a*^{+/+}/*Adam6b*^{-/+}) and homozygous (*Adam6a*^{+/+}/*Adam6b*^{-/-}) TRN0010 mice are herein referred to as heterozygous TRN0048 and homozygous TRN0048 mice, respectively.

*Pronuclear injection.* After administration of hormones, superovulated TRN0010 females were mated with TRN0010 males. One cell stage fertilized embryos were isolated from the oviducts at dpc 0.5. For microinjection, the one cell stage embryos were placed in a drop of M2 medium under mineral oil. A microinjection pipette with an approximate internal diameter of 0.5 micrometer (at the tip) was used to inject the mixed nucleotide preparation (see below) into the pronucleus of each embryo. After recovery, 25 - 35 injected one cell stage embryos were transferred to one of the oviducts of 0.5 dpc, pseudopregnant NMRI strain females.

*Mixed nucleotide preparation (MNP).* The MNP consisted of trans-activating CRISPR RNA (tracrRNA), gRNA1, gRNA2, and Cas9 protein. The Cas9 nuclease used for these pronuclear injections did not contain a nuclear localization signal (NLS).

### Example 2: Confirmation of Adam6b gene disruption by genomic PCR.

Genomic DNA was extracted from biopsies and analyzed by PCR (below). The following templates were used as controls: H₂O (no template) and wildtype genomic DNA (positive control). The PCR amplicons were analyzed by using a Caliper LabChip GX device. The locations of the PCR primers used in this analysis are shown in Figure 3 (Oligo 1, SEQ ID NO:9, Oligo 2, SEQ ID NO:10 and Oligo 3, SEQ ID NO:11). The fragment amplified with Oligos 1 and 2 detected the deleted (by non-homologous end joining) Adam6 locus. The unmodified wild type locus on the second TRN001 allele and potential indel (see below) modifications at the proximal gRNA cleavage site were detected by Oligos 1 and 3. Genomic PCR conditions were as follows:

### Reaction

5 µl PCR Buffer 10X (Invitrogen)
2 µl MgCl₂ (50mM)
1 µl dNTPs (10mM)
2 µl Primer 1 (SEQ ID NO:9, 5µM)
1 µl Primer 2 (SEQ ID NO:10, 5µM) OR 1 µl Primer 3 (SEQ ID NO:11, 5µM)
0.2 µl Taq (5U/µl, Invitrogen)
36.8 µl H₂O
2 µl DNA template
50 µl total volume

### Program

**95°C 5'**
**95°C 30"**
**60°C 30"**
**72°C 1'**
**72°C 10'**
35 cycles

### Expected Fragments (bp)

265 (*Adam6b* deletion. The exact size depended on how the locus was modified)
467 (*Adam6b* WT)
467 (*Adam6b* indel. The exact size depends on the extent of the indel)

Indels and heteroduplex formation. Indels are short insertions or deletions in genomic DNA. To distinguish indel modifications that do not result in deletion of *Adam6b* from the TRN0010 allele and unmodified wildtype sequences, a heteroduplex analysis via capillary electrophoresis was performed on *Adam6b* PCR products. Heteroduplex formation can occur during mixed-template polymerase chain reaction (PCR) using one pair of primers. A heteroduplex is a DNA double helix formed by annealing single strands of slightly different sequences. e.g., in this case *Adam6b* PCR products from TRN0010 indel and WT alleles, resulting in the formation of secondary structures, i.e. loops due to unpaired regions. Such loops may influence the migration of the amplicon during the capillary electrophoresis. Mice with indels near the *Adam6b* gene were identified by this analysis and excluded from further use.

Indels could occur at other regions of the TRN0010 genome as could off-target mutations. The frequency of such mutations *in vivo* has been difficult to determine precisely. There have been reports indicating a very low frequency in zygotes [Iyer, V, et al., Nat. Methods (2015) 12:479], but another report using PCR to amplify and then sequence predicted off-target sites indicated that off-target mutations are common *in vivo* [Aryal, NK, et al., Cell Death and Disease (2018) 9:1099-1101].

In any case, it is highly unlikely that any hypothetical off-target mutations in the TRN0048 *Adam6b* gene-deleted mice are responsible for the observed normal fertility of heterozygous TRN0048/TRN0001 male mice (see below) with only one copy of the *Adam6a* gene. For this to be the case, such mutated gene(s), which in their unmutated state have no male fertility restoring capabilities otherwise the *Adam6a*/*6b* KO would be fertile, would have to complement the ADAM6b deficiency.

### Example 3: Disruption of the Adam6a gene in TRN0010 zygotes using CRISPR/Cas9.

*Overview of the Adam6a targeting strategy.* According to NCBI and Ensembl databases, only one transcript exists for the *Adam6a* gene. The targeting strategy is based on NCBI transcript NM_174885.3, which corresponds to Ensembl transcript ENSMUST00000053086.2. Figure 6 illustrates the location of the *Adam6a* and *Adam6b* genes within the TRN0010 *Igh* locus (top) and the expected configuration of the locus after deletion of the *Adam6a* gene (bottom). Also indicated are the locations of the guide RNA binding sites, proximal (gRNA3, SEQ ID NO:12) and distal (gRNA1, SEQ ID NO:13) that were used in the CRISPR/Cas9-mediated deletion of *Adam6a.* These two guide RNAs were selected based on their position relative to *Adam6a* and the low number of potential off-targets. There were no sequences present in the TRN0010 mouse genome that were 100% identical to gRNA3 or gRNA1. The off-target analysis was based on NCBI BLASTn analysis of the GRCm38/mm10 mouse genome assembly [UCSC Genome Browser assembly ID: mm10; Sequencing/Assembly provider ID: Genome Reference Consortium Mouse Build 38 (GCA_000001635.2); Assembly date: Dec. 2011; Accession ID: GCA_000001305.2] and the TRN0010 *Igh* allele sequence. This targeting strategy allowed for the generation of constitutive knockout of the *Adam6a* gene within the TRN0010 *Igh* locus via CRISPR/Cas9-mediated gene editing. Heterozygous (*Adam6a*^{-/+}/*Adam6b*^{+/+}) and homozygous (*Adam6a*^{-/-}/*Adam6b*^{+/+}) TRN0010 mice are herein referred to as heterozygous TRN0059 and homozygous TRN0059 mice, respectively.

*Pronuclear injection.* After administration of hormones, superovulated TRN0010 females were mated with TRN0010 males. One cell stage fertilized embryos were isolated from the oviducts at dpc 0.5. For microinjection, the one cell stage embryos were placed in a drop of M2 medium under mineral oil. A microinjection pipette with an approximate internal diameter of 0.5 micrometer (at the tip) was used to inject the mixed nucleotide preparation (see below) into the pronucleus of each embryo. After recovery, 25 - 35 injected one cell stage embryos were transferred to one of the oviducts of 0.5 dpc, pseudopregnant NMRI strain females.

*Mixed nucleotide preparation (MNP).* The MNP consisted of trans-activating CRISPR RNA (tracrRNA), gRNA3, gRNA1, and Cas9 protein. The Cas9 nuclease used for these pronuclear injections did not contain a nuclear localization signal (NLS).

### Example 4: Confirmation of Adam6a gene disruption by genomic PCR.

Genomic DNA was extracted from biopsies and analyzed by PCR (below). The following templates were used as controls: H₂O (no template) and wildtype genomic DNA (positive control). The PCR amplicons were analyzed by using a Caliper LabChip GX device. The locations of the PCR primers used in this analysis are shown in Figure 4, Oligo 1, SEQ ID NO:14, Oligo 2, SEQ ID NO:15 and Oligo 3, SEQ ID NO:16. The fragment amplified with Oligos 1 and 2 detects the deleted (by non-homologous end joining) Adam6a locus. The unmodified wild type locus on the second TRN001 allele and potential indel modifications (see below) at the proximal gRNA cleavage site are detected by Oligos 1 and 3. Genomic PCR conditions were as follows:

### Reaction

5 µl PCR Buffer 10X (Invitrogen)
2 µl MgCl₂ (50mM)
1 µl dNTPs (10mM)
2 µl Primer 1 (SEQ ID 14, 5µM)
1 µl Primer 2 (SEQ ID 15, 5µM) OR 1 µl Primer 3 (SEQ ID 16, 5µM)
0.2 µl Taq (5U/µl, Invitrogen)
36.8 µl H₂O
2 µl DNA template
50 µl total volume

### Program

**95°C 5'**
**95°C 30"**
**60°C 30"**
**72°C 1'**
35 cycles
**72°C 10'**

### Expected Fragments (bp)

276 (*Adam6b* deletion. The exact size depended on how the locus was modified)
517 (*Adam6b* WT)
517 (*Adam6b* indel. The exact size depended on the extent of the indel)

*Indels and heteroduplex formation.* Indels are short insertions or deletions in genomic DNA. To distinguish indel modifications that do not result in deletion of *Adam6a* from the TRN0010 allele and unmodified wildtype sequences, a heteroduplex analysis via capillary electrophoresis was performed on *Adam6a* PCR products. Heteroduplex formation can occur during mixed-template polymerase chain reaction (PCR) using one pair of primers. A heteroduplex is a DNA double helix formed by annealing single strands of slightly different sequences. e.g., in this case *Adam6a* PCR products from TRN0010 indel and WT alleles, resulting in the formation of secondary structures, i.e. loops due to unpaired regions. Such loops may influence the migration of the amplicon during the capillary electrophoresis. Mice with indels near the *Adam6a* gene were identified by this analysis and excluded from further use.

Indels could occur at other regions of the TRN0010 genome as could off-target mutations. The frequency of such mutations *in vivo* has been difficult to determine precisely. There have been reports indicating a very low frequency in zygotes [Iyer, V, et al., Nat. Methods (2015) 12:479], but another report using PCR to amplify and then sequence predicted off-target sites indicated that off-target mutations are common in vivo [Aryal, NK, et al., Cell Death and Disease (2018) 9:1099-1101].

In any case, it is highly unlikely that any hypothetical off-target mutations in the TRN0059 *Adam6a* gene-deleted mice are responsible for the observed normal fertility of heterozygous TRN0059/TRN0001 male mice (see below) with only one *Adam6b* gene. For this to be the case, such mutated gene(s), which in their unmutated state have no male fertility restoring capabilities otherwise the *Adam6a*/*6b* KO would be fertile, would have to complement the ADAM6a deficiency.

### Example 5: Breeding strategy

Once mice were identified in which the *Adam6a* (TRN0059) or *Adam6b* (TRN0048) genes had been deleted on one TRN0010 allele, they were bred to TRN0001 mice, which have no *Adam6a*/*b* genes in their genome since these genes were deleted when the endogenous mouse *Ighv* locus was replaced with a humanized *IGHV* locus, i.e., they have the genotype *Adam6a⁰*^{/}*⁰*/*Adam6b⁰*^{/}*⁰*. The synthetic DNA construct containing the partly human *IGHV* locus that was introduced by RMCE into ES cells in which the endogenous mouse *Ighv* locus, including the *Adam6a*/*b* genes, had been deleted, did not include any *Adam6* genes. Male TRN0001 mice are thus infertile.

This breeding strategy allowed for rapid analysis of *Adam6a*/*b* mRNA expression and male fertility studies since these could be performed on F1 mice of the genotypes *Adam6a*^{*+*/*0*}/*Adam6b⁻* ^{/}*⁰* [from TRN0048 X TRN0001 matings] or *Adam6a⁻* ^{/}*⁰*/*Adam6b*^{+/*0*} [from TRN0059 X TRN0001 matings].

### Reference Example 6: Expression of Adam6a transcripts in wild type and Adam6 mutant mice.

Expression of *Adam6a* in WT (*Adam6a*^{+/+}/*Adam6b*^{+/+}), Adam6a heterozygous TRN0048/TRN0001 (*Adam6a*^{*+*/*0*}/*Adam6b*^{-/*0*}) and *Adam6a* null TRN0059/TRN0001 (*Adam6a*^{*-*/*0*}/*Adam6b*^{*+*/*0*}) mutant mice was analyzed by qPCR using the following protocol.

### Total RNA extraction:

Tissue disruption was carried out using the TissueLyser (QIAGEN) according to the **manufacturer's protocol.** Extraction of RNA was done with an RNeasy Plus Mini Kit **(QIAGEN) and the QiaCube Minirobot according to the manufacturer's protocol.** RNA concentration and 260/280 Ratio was determined using a Nanodrop ND-2000 UV-Vis spectrophotometer.

### cDNA Synthesis:

cDNA synthesis was performed with the High Capacity cDNA Reverse Transcription **Kit (Fisher Scientific GmbH) according to the manufacturer's protocol.** The input of RNA was standardized to 1000ng per 50µL sample.

### TaqMan Run:

Expression was measured on TaqMan 7900HT Fast Real-Time PCR System (Applied Biosystems, 384 well format) and the primers and probe shown at SEQ ID NO:17-19. TaqMan Assays and Gene Expression Master Mix were used in a reaction volume of 10µL. 2µL cDNA were used for each well, each sample was measured in triplicates. Results were normalized with a housekeeping Gene Expression Assay, Heterochromatin Protein 1 Binding Protein 3.

**Thermal profile:**

| | |
|---|---|
| Stage 1: | 95°C - 10 min |
| Stage 2: | 95°C - 15 sec |
| | 60°C - 1 min |
| | 40 Cycles |

Relative *Adam6a* expression in the testes of WT, null (TRN0059) and heterozygous (TRN0048) mice is shown in Figure 7. Each bar represents the expression value of a mouse tissue sample compared to the first wildtype mouse tissue sample (#2178063). As expected, there was no *Adam6a* qPCR signal in the TRN0059/TRN0001 *Adam6a* null sample. The relative expression in the TRN0048/TRN0001 *Adam6a* heterozygous mice was ~50% of wild type since these mice have only one copy of the *Adam6a* gene. These data are quantified in Figure 8. **The column labeled "Heavy" refers to the allele designation** for the *Adam6a* and *Adam6b* and human *IGH* genes on chromosome 12. HUM, human immunoglobulin V_{H}, D_{H} and J_{H} gene segment coding sequences embedded in mouse *Igh* flanking and regulatory sequences.

### Example 7: Expression of Adam6b transcripts in wild type and Adam6 mutant mice.

Expression of *Adam6b* in WT (*Adam6a*^{+/+}/*Adam6b*^{+/+}), heterozygous TRN0059/TRN0001 (*Adam6a⁻* ^{/}*⁰*/*Adam6b⁺* ^{/}*⁰*) and *Adam6b* null TRN0048/TRN0001 (*Adam6a⁺* ^{/}*⁰*/*Adam6b⁻* ^{/}*⁰*) mutant mice was analyzed by qPCR using the primers and probes shown at SEQ ID NO:20-22. Relative *Adam6b* expression in the testes of WT, heterozygous (TRN0059/TRN0001) and null (TRN0048/TRN0001) mice is shown in Figure 9. Each bar represents the expression value of a mouse tissue sample compared to the first wildtype mouse tissue sample (#2178063). The relative expression in the TRN0059/TRN0001 *Adam6b* heterozygous mouse was ~50% of wild type since this mouse has only one copy of the *Adam6b* gene. As expected, there was no *Adam6b* qPCR signal in the TRN0048/TRN0001 *Adam6a* null samples. These data are quantified in Figure 10. The **column labeled "Heavy" refers to the allele** designation for the *Adam6a* and *Adam6b* and human IGH genes on chromosome 12. HUM, human immunoglobulin V_{H}, D_{H} and J_{H} gene segment coding sequences embedded in mouse *Igh* flanking and regulatory sequences.

### Example 8: Fertility studies of male mice with only one Adam6 gene, Adam6a or Adam6b.

Heterozygous Adam6a TRN00048/TRN0001 (*Adam6a⁺* ^{/}*⁰*/*Adam6b⁻* ^{/}*⁰*) or *Adam6b* TRN0059/TRN0001 (*Adam6a⁻* ^{/}*⁰*/*Adam6b*^{+/*0*}) male mice were allowed to mate with wild type C57BL/6 female mice. The number of litters, the total number of pups born and the average litter size are shown in Table 1.

**Table 1. Fertility data for hemizygous Adam6a and Adam6b male mice compared to WT. Females were WT C57BL/6.**

| **Male designation** | **Adam6 genotype** | **Number of litters** | **Total number of viable pups** | **Average litter size** |
|---|---|---|---|---|
| TRN0048/TRN0001 | (*Adam6a⁺* ^{/}*⁰*/ *Adam6b⁻* ^{/}*⁰*) | 10 | 77 | 7.7 |
| C57BL/6 WT* | (*Adam6a⁺* ^{/}*⁺*/ *Adam6b*^{*+*/*+*}) | | | 5-6 |
| TRN0059/TRN0001 | (*Adam6a⁻* ^{/}*⁰*/ *Adam6b⁺* ^{/}*⁰*) | 4 | 21 | 5.25 |
| *Historical data from Charles River Laboratories, where the TRN0001, TRN0048, TRN0059 and respective F1 mice were housed. | | | | |

These results show that the fertility of the TRN0048/TRN0001 and TRN0059/TRN0001 male mice was comparable to WT C57BL/6 male mice. This is despite the fact that the TRN0048/TRN0001 mice and the TRN0059/TRN0001 mice have only one *Adam6* gene, namely *Adam6a* or *Adam6b* respectively, whereas the WT mice have four *Adam6* genes, namely two *Adam6a* and two *Adam6b* genes. Thus, contrary to previous reports and to expectation, only one *Adam6* gene, *Adam6a* or *Adam6b,* is required for full male fertility in mice.

## Claims

1. A transgenic mouse that is engineered by a deletion of endogenous *Adam6a* and *Adam6b* genes in a male mouse, wherein the mouse is homozygous for the deletion, and comprises in its genome only one exogenous *Adam6* transgene, and expresses an ADAM6 protein comprising at least 90% sequence identity to SEQ ID NO:2, which *Adam6* transgene is functional in a male mouse,
wherein the mouse comprises an immunoglobulin heavy chain variable region (IHVR) locus comprising human immunoglobulin gene segment (hIGS) coding sequences and murine expression control sequences in operable linkage to control expression of the hIGS coding sequences, and wherein the exogenous *Adam6* transgene is embedded in said IHVR locus,
wherein said hIGS coding sequences and said murine expression control sequences are comprised in a heterologous expression cassette further comprising said *Adam6* transgene, wherein said heterologous expression cassette is integrated within the mouse genome and replaces endogenous immunoglobulin gene segment coding sequences, or replaces an immunoglobulin gene segment of the endogenous IHVR locus, and
wherein said *Adam6* transgene is *Adam6b.*

2. The mouse of claim 1, which comprises in its genome immunoglobulin gene segments that upon VDJ rearrangement encode immunoglobulin sequences of one or more antibody variable domains.

3. Use of the mouse of claim 2 in a method for the production of antibodies.

4. A recombinant expression cassette comprising human immunoglobulin gene segment (hIGS) coding sequences and murine expression control sequences in operable linkage to control expression of the hlGS coding sequences, and only one *Adam6* transgene capable of expressing an ADAM6 protein comprising at least 90% sequence identity to SEQ ID NO:2, which Adam6transgene is functional in a male mouse, wherein said *Adam6* transgene is *Adam6b.*

5. The expression cassette of claim 4, comprising said murine expression control sequences as intergenic sequences embedded in a gene segment comprising the hlGS coding sequences.

6. The expression cassette of claim 4 or 5, comprising
a) human V_{H} gene segment coding sequences including heavy chain variable region gene segment (*IGHV*) coding sequences, diversity gene segment *(IGHD)* coding sequences, and joining gene segment (*IGHJ*) coding sequences; and
b) said *Adam6* transgene embedded between the *IGHV* and the *IGHD* coding sequences, preferably in opposite transcriptional orientation to the *IGHV* gene segment coding sequences.

7. A method for producing a mouse of claim 1 or 2, comprising:
a) providing an embryonic mouse stem cell;
b) providing one or more vectors comprising at least one expression cassette containing gene segments that upon VDJ rearrangement encode immunoglobulin sequences;
c) introducing said one or more vectors into the cell;
d) incorporating said gene segments into the genome of the cell, and selecting a transgenic cell wherein said gene segments have been integrated into the cellular genome of said transgenic cell at a target site at the endogenous immunoglobulin heavy chain gene locus, thereby deleting endogenous *Adam6a* and *Adam6b* genes; and
e) utilizing said transgenic cell to create a transgenic mouse comprising said transgenic cell;
wherein at least one of said vectors comprises the expression cassette of any one of claims 4 to 6.

## Patentansprüche

1. Transgene Maus, die durch eine Deletion von endogenen *Adam6a-* und Adam6b-Genen in einer männlichen Maus gentechnisch erzeugt wird, wobei die Maus homozygot für die Deletion ist und in ihrem Genom nur ein exogenes Adam6-Transgen umfasst, und ein ADAM6-Protein umfassend mindestens 90 % Sequenzidentität mit SEQ ID NO:2 exprimiert, wobei das Adam6-Transgen in einer männlichen Maus funktional ist,
wobei die Maus einen Lokus für die variable Immunglobulin-Region der schweren Kette (IHVR) umfassend für humanes Immunglobulin-Gensegment (hIGS) kodierende Sequenzen und murine Expressionssteuersequenzen in Wirkverbindung umfasst, um die Expression der für hIGS kodierenden Sequenzen zu steuern, und wobei das exogene Adam6-Transgen in den IHVR-Lokus eingebettet ist,
wobei die für hlGS kodierenden Sequenzen und die murinen Expressionssteuersequenzen in einer heterologen Expressionskassette umfasst sind, die ferner das Adam6-Transgen umfasst, wobei die heterologe Expressionskassette in das Maus-Genom integriert ist und endogene für das Immunglobulin-Gensegment kodierende Sequenzen ersetzt oder ein Immunglobulin-Gensegment des endogenen IHVR-Lokus ersetzt, und wobei das Adam6-Transgen *Adam6b* ist.

2. Maus nach Anspruch 1, welche in ihrem Genom Immunglobulin-Gensegmente umfasst, die nach VDJ-Umordnung für Immunglobulin-Sequenzen von einer oder mehreren variablen Antikörper-Domänen kodieren.

3. Verwendung der Maus nach Anspruch 2 in einem Verfahren für die Herstellung von Antikörpern.

4. Rekombinante Expressionskassette, umfassend für humanes Immunglobulin-Gensegment (hIGS) kodierende Sequenzen und murine Expressionssteuersequenzen in Wirkverbindung, um die Expression der für hIGS kodierenden Sequenzen zu steuern, und nur ein Adam6-Transgen, das in der Lage ist, ein ADAM6-Protein umfassend mindestens 90 % Sequenzidentität mit SEQ ID NO:2 zu exprimieren, wobei das *Adam6-*Transgen in einer männlichen Maus funktional ist, wobei das Adam6-Transgen *Adam6b* ist.

5. Expressionskassette nach Anspruch 4, umfassend die murinen Expressionssteuersequenzen als Intergen-Sequenzen, die in ein Gensegment umfassend die für hIGS kodierenden Sequenzen eingebettet sind.

6. Expressionskassette nach Anspruch 4 oder 5, umfassend
a) für das humane VH-Gensegment kodierende Sequenzen, die für das Gensegment der variablen Region der schweren Kette *(IGHV)* kodierende Sequenzen, für das Diversitäts-Gensegment *(IGHD)* kodierende Sequenzen, und für das verbindende Gensegment (*IGHJ*) kodierende Sequenzen beinhalten; und
b) wobei das Adam6-Transgen zwischen den für *IGHV* und den für *IGHD* kodierenden Sequenzen eingebettet ist, vorzugsweise in zu den für das *IGHV-*Gensegment kodierenden Sequenzen gegenläufiger transkriptioneller Orientierung.

7. Verfahren zur Herstellung einer Maus nach Anspruch 1 oder 2, umfassend:
a) Bereitstellen einer embryonalen Mausstammzelle;
b) Bereitstellen eines oder mehrerer Vektoren umfassend mindestens eine Expressionskassette enthaltend Gensegmente, die nach VDJ-Umordnung für Immunglobulin-Sequenzen kodieren;
c) Einführen des einen oder der mehreren Vektoren in die Zelle;
d) Einbeziehen der Gensegmente in das Genom der Zelle, und Auswählen einer transgenen Zelle, bei welcher die Gensegmente in das zelluläre Genom der transgenen Zelle an einer Zielstelle an dem Lokus des Gens für die endogene schwere Kette von Immunoglobulin integriert wurden, wodurch endogene *Adam6a-* und Adam6b-Gene deletiert werden; und
e) Verwenden der transgenen Zelle, um eine transgene Maus zu schaffen, welche die transgene Zelle umfasst;
wobei mindestens einer der Vektoren die Expressionskassette nach einem der Ansprüche 4 bis 6 umfasst.

## Revendications

1. Souris transgénique qui est modifiée par une délétion des gènes *Adam6a* et *Adam6b* endogènes chez une souris mâle, la souris étant homozygote pour la délétion, et comprend dans son génome un seul transgène *Adam6* exogène, et exprime une protéine ADAM6 comprenant au moins 90 % d'identité de séquence avec la SEQ ID NO:2, lequel transgène *Adam6* est fonctionnel chez une souris mâle,
la souris comprenant un locus de région variable de chaîne lourde d'immunoglobuline (IHVR) comprenant des séquences codantes de segment de gène d'immunoglobuline humaine (hIGS) et des séquences de régulation d'expression murines en liaison fonctionnelle pour réguler l'expression des séquences codantes de hIGS, et dans laquelle le transgène *Adam6* est incorporé dans ledit locus d'IHVR,
dans laquelle lesdites séquences codantes de hIGS et lesdites séquences de régulation d'expression murines sont comprises dans une cassette d'expression hétérologue comprenant en outre ledit transgène *Adam6,* dans laquelle ladite cassette d'expression hétérologue est intégrée dans le génome de souris et remplace les séquences codantes de gène d'immunoglobuline endogène, ou remplace un segment de gène d'immunoglobuline du locus d'IHVR endogène, et dans laquelle ledit transgène *Adam6* est *Adam6b.*

2. Souris selon la revendication 1, qui comprend dans son génome des segments de gène d'immunoglobuline qui lors d'un réarrangement VDJ codent des séquences d'immunoglobuline d'un ou plusieurs domaines variables d'anticorps.

3. Utilisation de la souris selon la revendication 2 dans un procédé de production d'anticorps.

4. Cassette d'expression recombinante comprenant des séquences codantes de segment de gène d'immunoglobuline humaine (hIGS) et des séquences de régulation d'expression murines en liaison fonctionnelle pour réguler l'expression des séquences codantes de hIGS, et un seul transgène Adam6 capable d'exprimer une protéine ADAM6 comprenant au moins 90 % d'identité de séquence avec la SEQ ID NO:2, lequel transgène *Adam6* est fonctionnel chez une souris mâle, dans laquelle ledit transgène *Adam6* est *Adam6b.*

5. Cassette d'expression selon la revendication 4, comprenant lesdites séquences de régulation d'expression murines sous forme de séquences intergéniques incorporées dans un segment de gène comprenant les séquences codantes de hIGS.

6. Cassette d'expression selon la revendication 4 ou 5, comprenant
a) des séquences codantes de segment de gène de V_{H} humain comportant des séquences codantes de segment de gène de région variable de chaîne lourde *(IGHV),* des séquences codantes de segment de gène de diversité *(IGHD)* et des séquences codantes de segment de gène de jonction *(IGHJ)* ; et
b) ledit transgène *Adam6* incorporé entre les séquences codantes *d'IGHV* et d'*IGHD,* de préférence dans une orientation transcriptionnelle opposée aux séquences codantes de segment de gène d'*IGHV.*

7. Procédé de production d'une souris selon la revendication 1 ou 2, comprenant :
a) la fourniture d'une cellule souche de souris embryonnaire ;
b) la fourniture d'un ou plusieurs vecteurs comprenant au moins une cassette d'expression contenant des segments de gène qui lors d'un réarrangement VDJ codent des séquences d'immunoglobuline ;
c) l'introduction desdits un ou plusieurs vecteurs dans la cellule ;
d) l'incorporation desdits segments de gène dans le génome de la cellule et la sélection d'une cellule transgénique dans laquelle lesdits segments de gène ont été intégrés dans le génome cellulaire de ladite cellule transgénique au niveau d'un site cible au niveau du locus de gène de chaîne lourde d'immunoglobuline endogène, d'où une délétion des gènes Adam6a et Adam6b endogènes ; et
e) l'utilisation de ladite cellule transgénique pour créer une souris transgénique comprenant ladite cellule transgénique ;
au moins l'un desdits vecteurs comprenant la cassette d'expression selon l'une quelconque des revendications 4 à 6.
